(19) Europäisches Patentamt
European Patent Office
Office européen des brevets

(11) Publication number: 0 373 216 A1

(12) **EUROPEAN PATENT APPLICATION published in accordance with Art. 158(3) EPC**

(21) Application number: 88901319.9

(22) Date of filing: 02.02.88

(86) International application number: PCT/JP88/00089

(87) International publication number: WO 88/05776 (11.08.88 88/18)

(51) Int. Cl.5: C07D 501/46, C07D 501/57, A61K 31/545

(30) Priority: 02.02.87 JP 20576/87

(43) Date of publication of application: 20.06.90 Bulletin 90/25

(84) Designated Contracting States: CH DE FR GB LI

(71) Applicant: TEIJIN LIMITED
11 Minamihonmachi 1-chome Higashi-ku
Osaka-shi Osaka 541(JP)

(72) Inventor: HARADA, Toshiaki
69-5, Ushinoya-machi 3-chome
Iwakuni-shi Yamaguchi 741(JP)
Inventor: YOSHISATO, Eishin
110-22, Minamiiwakuni-machi 2-chome
Iwakuni-shi Yamaguchi 740(JP)
Inventor: IMAI, Hiroshi
9-2, Yamate-cho 2-chome
Iwakuni-shi Yamaguchi 740(JP)
Inventor: TAKANO, Yasunobu
9-2, Yamate-cho 2-chome
Iwakuni-shi Yamaguchi 740(JP)
Inventor: ICHIKAWA, Yataro
11-7, Kotesashi-machi 2-chome
Tokorozawa-shi Saitama 359(JP)
Inventor: SUZUKI, Yoji
20-2, Tamadaira 5-chome
Hino-shi Tokyo 191(JP)

(74) Representative: Myerscough, Philip Boyd et al
J.A.Kemp & Co. 14, South Square Gray's Inn
London, WC1R 5EU(GB)

(54) **CEPHALOSPORIN COMPOUNDS OR THEIR SALTS, PROCESS FOR THEIR PREPARATION, AND PARMACEUTICAL COMPOSITIONS.**

(57) Cephalosporin compounds represented by general formula (I) or their salts, a process for their preparation, and antibacterial agents containing them as active ingredients, wherein Y represents an optionally protected amino group; Z represents a hydrogen atom, a methoxy group or a formamino group; $R^1$ represents an optionally substituted alkyl group or a hydrogen atom; $R^2$, and $R^3$, which may

EP 0 373 216 A1

be the same or different, each represents a hydrogen atom or a hydroxy group protecting group; W represents a substituent; and n represents an integer of 0 to 5.

TITLE MODII
see front page



SPECIFICATION

Cephalosporin compounds or their salts, process for production of same and their pharmaceutical compositions

## BACKGROUND OF INVENTION

### 1. Field of Invention

This invention relates to a novel cephalosporin compound or its salt, process for the production of same and its pharmaceutical composition. More specifically, this invention relates to a novel cephalosporin compound having a 6,7-dihydroxyisoquinoliniummethyl group or its dihydroxyl group as a substituent in the 3-position or its pharmaceutically acceptable salt, a process for the production of same, and its pharmaceutical composition. The Cephalosporin compound and its pharmaceutically acceptable salt in this invention have very high antibacterial activity and strong activity capable of hindering growth of wide-ranging Gram-positives and Gram-negatives, especially showing excellent antibacterial activity to Gram-negatives, above all, Pseudomonas aeruginosa.

### 2. Description of Prior Art

Cephalosporin compounds having e.g. a 2-substituted imino-2-(2-amino-1,3-thiazol-4-yl)acetamide group in the 7-position of a cephalosporin skeleton are known as compounds having antibacterial properties, and varied compounds shown below have been already proposed, for example.

(i) Japanese Patent Publication No. 58353/1983 (British Patent No. 1536281)

The cephalosporin compounds described in this document are principally compounds having a 2-substituted imino-2-(2-amino-1,3-thiazol-4-yl)acetamide group in the 7-position and a substituted methyl group of a nucleophilic compound residue in the 3-position.

(ii) Japanese Laid-open Patent Application No. 57386/ 1983 (U.S. Patent No. 4396619)

This document proposes cephalosporin compounds having a group typified by a 2-substituted imino-2-(2-amino-1,3-thiazol-4-yl)acetamide in the 7-position and an isoquinolinium methyl group that may have a specific substituent in the 3-position.

(iii) Japanese Laid-open Patent Application No. 130294/1984 (European Patent No. 111935)

This document discloses cephalosporin compounds having a 2-substituted imino-2-(2-amino-1,3-thiazol-4-yl)-acetamide group in the 7-position and a quinoliniummethyl group or an isoquinoliniummethyl group that may have a specific substituent in the 3-position.

3. Objects and Outline of Invention

It is an object of this invention to provide a novel cephalosporin compound having a 6,7-dihydroxyisoquinoliniummethyl group or its dihydroxyl group as a substituent in the 3-position and a specific substituent in the 7-position.

Another object of this invention is to provide a novel cephalosporin compound having wide-ranging antibacterial spectrum and high antibacterial activity.

Still another object of this invention is to provide a novel cephalosporin compound having very high antibacterial activity to Gram-positives and Gram-negatives.

Yet another object of this invention is to provide a novel cephalosporin compound having very high antibacterial activity to Gram-negatives such as Pseudomonas aeruginosa, Salmonella typhi, Enterobacter, etc. in particular.

A further object of this invention is to provide a novel cephalosporin compound having excellent water-solubility and low toxicity.

A still further object of this invention is to provide a process for the production of a novel cephalosporin compound and its phamaceutical use.

The other objects and advantages of this invention will be made clear from the following description.

According to studies of this invention, it is found that these objects and advantages are achieved by the following cephalosporin compound.

Namely, this invention provides a cephalosporin compound represented by formula (I)

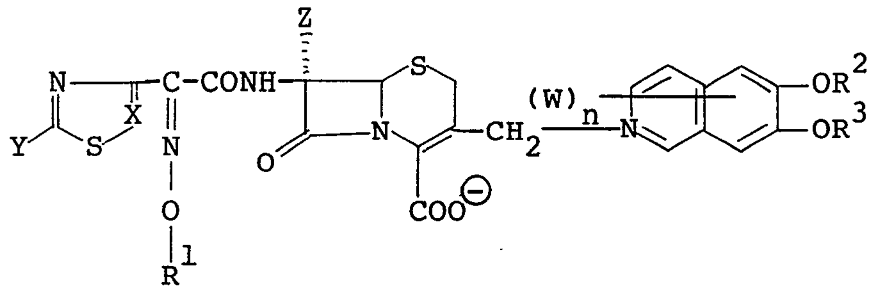

wherein

X denotes -CH= or -N=,

Y denotes an amino group that may be protected,

Z denotes a hydrogen atom, a methoxy group or a formylamino group,

$R^1$ denotes an aromatic hydrocarbon group that may be substituted,

$R^2$ and $R^3$ are the same or different, and each denotes a hydrogen atom or a protective group of a hydroxyl group,

W denotes a substituent, and

n is an integer of 0 to 5,

or its pharmaceutically acceptable salt.

One of the characteristics of such cephalosporin compound in this invention is that it has an isoquinoline skeleton of the following structure in the 3-position.

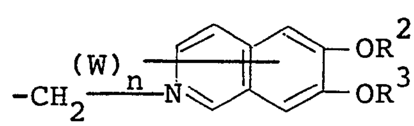

The isoquinoline skeleton has at least hydroxyl groups or protected hydroxyl groups indicated by $-OR^2$ and $-OR^3$ in the 6- and 7-positions.

Another characteristic of the cephalosporin compound in this invention is that it has an acetamide group of the following structure in the 7-position.

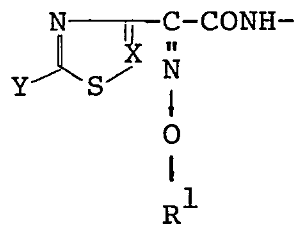

The acetamid group is characterized in that it has an imino group wherein $R^1$ is an aromatic hydrocarbon group that may be substitute, and further a 2-amino (or protected amino)-1,3-thiadiazol-4-yl group or a 5-amino (or protected amino)-1,2,4-thiadiazol-3-yl group.

The cephalosporin compound of this invention with the above characteristics has such excellent physiological activity that it possesses wide-ranging antibacterial spectrum and very high antibacterial activity. Especially, it possesses wide-ranging antibacterial spectrum and very high antibacterial activity to Gram-negatives, and can hinder growth of bacteria in a surprisingly small amount. Particularly noteworthy is that the cephalosporin compound shows a higher activity to Pseudomonas aeruginosa than hitherto attainable with the commercial cephalosporin compounds.

Moreover, becuase $R^1$ is an aromatic hydrocarbon group, it provides also the characteristics, compared to $R^1$ of another alkyl or substituted alkyl, that it has wide-ranging antibacterial spectrum to Gram-positives and high antibacterial activity.

4. <u>Description of Preferable embodiments of Invention</u>

The cephalosporin compound (I) of this invention is explained in more detail below.

The cephalosporin compound represented by formula (I) in this invention has partially a 2-amino-1,3-thiazol-4-yl skeleton or a 5-amino-1,2,4-thiadiazol-3-yl skeleton reprsented by formula:

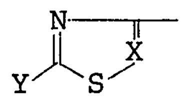

This skeleton can easily be isomerized to a 2-amino-thiazolin-4-yl skeleton or a 5-aminothiadiazolin-3-yl skeleton represented by formula:

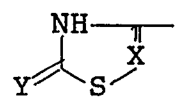

In this invention, either interchangeable isomer or a mixture of these isomers will do.

Moreover, the cephalosporin compound of formula I in this invention has partially an imino group represented by formula:

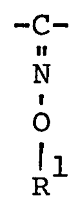

The imino group moiety to which $R^1$-O- is bound includes a syn-form and an anti-form. Of these, the syn-form is preferable becuase of the higher antibacterial activity.

Y in formula (I) is an amino group that may be protected, and therefore means an amino group itself or an amino group protected with a protective group. In case of the protected amino group, the protective group is preferably an ordinary protective group of an amino group. Examples of the protective group are an acyl

group that may be substituted, an aralkyl group that may be substituted, and a silyl group that may be substituted. Examples of such acyl group are lower alkanoyl groups such as formyl, acetyl, propionyl, butyryl, iso-butyryl, valeryl, iso-valeryl, oxalyl, succinyl, pivaloyl and trityloxyacetyl; lower alkoxycarbonyl groups such as methoxycarbonyl, ethoxycarbonyl, propoxycarbonyl, isopropoxycarbonyl, butoxycarbonyl and pentyloxycarbonyl; lower alkanesulfonyl groups such as mesyl, ethanesulfonyl, propanesulfonyl, iso-propanesulfonyl and butanesulfonyl; aroyl groups such as benzoyl, toluoyl, naphthoyl and phthaloyl; aralkanoyl groups such as phenylacetyl and phenylpropionyl; and aralkoxycarbonyl groups such as benzoyloxycarbonyl. Such acyl group may have one or more suitable substituents. Examples of the substituent are a halogen atom such as chlorine, bromine, iodine or fluorine, a cyano group, and a lower alkyl group such as methyl, ethyl, propyl or butyl. Examples of the aralkyl group that may have a substituent are benzyl, 4-methoxybenzyl, phenetyl, trityl, methoxytrityl, dimethoxytrityl and 3,4-dimethoxybenzyl.

Examples of the silyl group that may be substituted are trimethylsilyl, dimethyl-t-butylsilyl, methyl-di-t-butylsilyl, triphenylsilyl and diphenyl-t-butylsilyl.

$R^1$ in formula (I) of the cephalosporin compound in this invention is an aromatic hydrocarbon group that may be substituted. The aromatic hydrocarbon group having 6 to 20 carbon atoms, especially 6 to 15 carbon atoms is preferable. Preferable examples of the aromatic hydrocarbon group indicated by $R^1$ are benzene, diphenyl, naphthalene, anthracene and 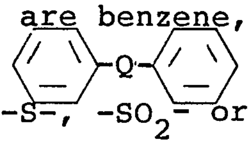 (wherein Q denotes -O-, $-\overset{\mathrm{O}}{\overset{\|}{\mathrm{C}}}-$, -S-, $-SO_2-$ or lower alkylene). Benzene is especially preferable.

Such aromatic hydrocarbon group may have a substituent in any position. When the aromatic hydro-

carbon group $R^1$ has a substituent, the number of the substituent may be 1 or more, preferably 1 to 10, most preferably 1 to 5. In case of 2 or more substituents, different substituents are also available. Examples of the substituent are an alkyl group with 1 to 4 carbon atoms, such as methyl, ethyl, n-propyl, iso-propyl, n-butyl, iso-butyl or tert-butyl; an alkoxy group with 1 to 4 carbon atoms such as methoxy, ethoxy, propoxy or butoxy; halogen such as fluorine, chlorine, bromine or iodine; an alkyl group with 1 to 4 carbon atoms which is substituted by halogen, a hydroxyl group, a carboxyl group or an amino group; an amino group; a hydroxyl group: a carboxyl group; a cyano group; a sulfo group; an aminosulfo group; a formyl group; a thiocarbamoyl group; a carbamoyl group with 2 to 4 carbon atoms; an alkylamino group with 1 to 4 carbon atoms; a di-(alkyl with 1 to 4 carbon atoms)amino group; a carbamoyloxy group; a thiocarbamoyl group; an acyloxy group with 2-4 carbon atoms; a sulfonamide group; and an alkoxycarbonyl group with 1 to 4 carbon atoms. However, these are not critical.

As $R^1$, a phenyl group or a substituted phenyl group is preferable. On this occasion, preferable examples of the substituent are halogen, an alkyl group with 1 to 4 carbon atoms substituted with halogen, a carboxyl group, a carbamoyl group, an alkoxy group with 1 to 4 carbon atoms and a group of formula, $(O)_l(CH_2)_mCOY'$ wherein l and m are, independently from each other, 0 or 1, and Y' denotes OH, $NH_2$ or an alkoxy group with 1 to 4 carbon atoms.

In the cephalosporin compound of this invention, X in formula (I) is -CH= or -N=, forming a thiazole ring or a thiadiazole ring. Either of them will do.

Z in formula I is a hydrogen atom, a methoxy group or a formylamino group. Of these, the hydrogen atom or the methoxy group is preferable. The hydrogen atom is most preferable.

As stated above, the cephalosporin compound of this invention has, as shown in formula (I), an iso quinoline skeleton having at least $-OR^2$ and $-OR^3$ in the 6- and 7-positions. $R^2$ and $R^3$ are, independently from each other, a hydrogen atom or a protective group of a hydroxyl group. The protective group of the hydroxyl group is a group convertible chemically or biologically into a hydroxyl group. Examples of such protective group are an acyl group, a silyl group, a phosphoric acid group, a phosphinic acid group, a sulfonic acid group, a sulfinic acid group and a sulfuric acid group. $R^2$ and $R^3$ may together form a ring with an oxygen atom to which said groups are bound. The acyl group is preferably an acyl group with 2 to 4 carbon atoms, and the carbon atoms of the silyl group may have further substituents such as a hydroxyl group, an amino group, a carboxyl group and a cyano group. Examples of the silyl group are trialkylsilyl groups such as a trimethylsilyl group, triethylsilyl group, a tert-butyldimethylsilyl group, a di-tert-butylmethylsilyl group and a tributylsilyl group; aryl-substituted silyl groups such as a diphenyl-tert-butylsilyl group; dialkylsilyl groups wherein $R^2$ and $R^3$ together form a ring, such as a di-tert-butylsilyl group, a diethylbutylsilyl group, and a di(di-tert-butylsiloxy) group.

The phosphoric acid group or the sulfuric acid group is a group forming a salt or an esteramide. Examples thereof are $-OPO(OH)_2$, $-OPO(OH)(ONa)$, $-OPO(OH)(OK)$, $-OPO(ONa)_2$, $-OPO(OK)_2$, $-OPO(OH)(OR^6)$, $-OPO(OR^6)_2$, $OPO(OR^6)(OR^7)$,

$-OPO(OH)(N-H_2)$, $-OPO(OH)(NHR^6)$, $-OPO(N\genfrac{}{}{0pt}{}{R^6}{R^7})_2$, $-OSO_2(OH)$,

$-OSO_2(OK)$, $OSO_2(ONa)$, $OSO_2NH_2$, $OSO_2-(NR^6R^7)$ and $OSO_2R^6$. In these examples of the phosphoric acid group or the sulfuric acid group, $R^6$ and $R^7$ may be the same or different, and each denotes an alkyl group with 1 to 4

carbon atoms that may be substituted.

Moreover, examples of $R^2$ and $R^3$ forming a ring together with an oxygen atom to which $R^2$ and $R^3$ are bound are $-OPO(R^9)O-$, $-OPO(OR^8)O-$, $OSO_2O-$, $-OCOO-$, $-OSi(R^9)_2O-$, $-OSi(R^9)_2-O-Si(R^9)_2-O-$, $OPO(OR^8)O(OR^8)OPO-$ and $OP-(OH)O-$. Where $R^2$ and $R^3$ form part of a ring, $R^8$ is a hydrogen atom, a metal ion, an ammonium ion that may be substituted, or an alkyl group that may be substituted or an aryl group that may be substituted. The phosphonic acid group is a group represented by formula -O-PO-(A)(OB) wherein A is an alkyl group that may be substituted or an aryl group that may be substituted, and B is a hydrogen atom, a metal ion, an ammonium ion that may be substituted, an alkyl group that may be substituted or an aryl group that may be substituted. Or it may form a ring with an adjacent hydrogen group to give -O-OP(A)-O-. The sulfonic acid group is one represented by formula $-O-S(O)_2A$ wherein A is an alkyl group or an aryl group that may be substituted.

The isoquinoline skeleton may have two substituents $-OR^2$ and $-OR^3$ in the 6- and 7-positions or further the other substituent $[(W)_n]$ in the other position. In this case, the number (n) of substituents is 0 to 5, preferably 0 to 2, and most preferably 0 to 1. Examples of the substituent (-W) may be varied substituents; it can be the same as those of $R^1$ shown above.

Examples of the substituent (-W) are an amino group, an alkylamino group with 1 to 4 carbon atoms, a di(alkyl with 1 to 4 carbon atoms)amino group, a hydroxyl group, an alkoxy group with 1 to 4 carbon atoms, a carbamoyl group, a carbamoyloxy group, a thiocarbamoyl group, an acyloxy group with 2 to 4 carbon atoms, a cyano gorup, a halogen atom, a halogenated alkyl group with 1 to 4 carbon atoms, a sulfo group, an aminosulfonyl group, an alkanoyl group with 2 to 4 carbon atoms, an alkyl group with 1 to 4 carbon atoms, a carboxyl group, an

alkoxycarbonyl group with 1 to 4 carbon atoms, a hydroxyl group, an alkyl group with 1 to 4 carbon atom, and a formyl group.

In the cephalosporin compound of formula (I) in this invention, a cation+ in the 3-position forms salts with varied anions. Examples of the anions are ions of monobasic acids such as $Cl^-$, $Br^-$, $HCOO^-$, $-CH_3COO^-$, $CCl_3COO^-$ and $CF_3COO^-$.

Other salts of the cephalosporin compound represented by formula (I) are salts of said compound having a carboxyl group ($-COO^-$) in the 4-position. Examples of the salts are alkali metal salts such as sodium and potassium salts, alkaline earth metal salts such as calcium and magnesium salts, ammonium salts, salts with organic bases such as triethylammonium, trimethylammonium, pyridinium, tetraethylammonium, tetra-n-butylammonium salts.

The cephalosporin compound of formula (I) may be an ester with the carboxyl group in the 4-position esterified. Such ester may be an ester that is easily hydrolyzed physiologically. Examples of such ester are alkoxyalkyl esters such as methoxymethyl, ethoxymethyl, methoxyethyl, ethoxyethyl esters; alkanoyloxyalkyl esters such as acetoxymethyl, propioxymethyl, butyryloxymethyl, valeryloxymethyl and pivaloyloxymethyl esters; and indanyl, phthalidyl, glycyloxymethyl and phenylglycyloxymethyl esters.

In this invention, the cephalosporin compound, its salt or its ester in crystalline form is chemically stable. Therefore, the cephalosporin compound, its salt or its ester in crystalline form is desirous when used as an active ingredient of a pharmaceutical composition. The crystals may be anhydrides or hydrates such as a 3/4-hydrate, a 1-hydrate, a 1,5-hydrate, a 3-hydrate and a 5-hydrate.

Preferable concrete examples of the cephalosporin compound in this invention are as follows.

(1) (6R,7R)-7-[(Z)-2-(2-amino-1,3-thiazol-4-yl)-2-phenoxyiminoacetamide]-3-(6,7-dihydroxy-2-isoquinolinium)methyl-3-cephem-4-carboxylate

(2) (6R,7R)-7-[(Z)-2-(2-amino-1,3-thiazol-4-yl)-2-(4-methyl-phenoxyimino)acetamide]-3-(6,7-dihydroxy-2-isoquinolinium)methyl-3-cephem-4-ca rboxylate

(3) (6R,7R)-7-[(Z)-2-(2-amino-1,3-thiazol-4-yl)-2-(3- methoxyphenoxyimino)acetamide]-3-(6,7-dihydroxy-2-isoquinolinium)methyl-3-cephem-4-carboxylate

(4) (6R,7R)-7-[(Z)-2-(2-amino-1,3-thiazol-4-yl)-2-(2-chlorophenoxyimino)acetamide]-3-(6,7-dihydroxy-2-isoquinolinium)methyl-3-cephem-4-carboxylate

(5) (6R,7R)-7-[(Z)-2-(2-amino-1,3-thiazol-4-yl)-2-(4-chlorophenoxyimino)acetamide]-3-(6,7-dihydroxy-2-isoquinolinium)methyl-3-cephem-4-carboxylate

(6) (6R,7R)-7-[(Z)-2-(2-amino-1,3-thiazol-4-yl)-2-(3-bromophenoxyimino)acetamide]-3-(6,7-dihydroxy-2-isoquinolinium)methyl-3-cephem-4-carboxylate

(7) (6R,7R)-7-[(Z)-2-(2-amino-1,3-thiazol-4-yl)-2-(2-chlorophenoxyimino)acetamide]-3-(6,7-dihydroxy-2-isoquinolinium)methyl-3-cephem-4-carboxylate

(8) (6R,7R)-7-[(Z)-2-(2-amino-1,3-thiazol-4-yl)-2-(4-chlorophenoxyimino)acetamide]-3-(6,7-dihydroxy-2-isoquinolinium)methyl-3-cephem-4-carboxylate

(9) (6R,7R)-7-[(Z)-2-(2-amino-1,3-thiazol-4-yl)-2-(3-carboxyphenoxyimino)acetamide]-3-(6,7-dihydroxy-2-isoquinolinium)methyl-3-cephem-4-carboxylate

(10) (6R,7R)-7-[(Z)-2-(2-amino-1,3-thiazol-4-yl)-2-(2-carboxymethylphenoxyimino)acetamide]-3-(6,7-dihydroxy-2-isoquinolinium)methyl-3-cephem-4-carboxylate

(11) (6R,7R)-7-[(Z)-2-(2-amino-1,3-thiazol-4-yl)-2-(4- carboxymethylphenoxyimino)acetamide]-3-(6,7-dihydroxy-2-isoquinolinium)methyl-3-cephem-4-carboxylate

(12) (6R,7R)-7-[(Z)-2-(2-amino-1,3-thiazol-4-yl)-2-(4-cyanomethylphenoxyimino)acetamide]-3-(6,7-dihydroxy-2-isoquinolinium)methyl-3-cephem-4-carboxylate

(13) (6R,7R)-7-[(Z)-2-(2-amino-1,3-thiazol-4-yl)-2-(4-hydroxymethylphenoxyimino)acetamide]-3-(6,7-dihydroxy-2-isoquinolinium)methyl-3-cephem-4-carboxylate

(14) (6R,7R)-7-[(Z)-2-(2-amino-1,3-thiazol-4-yl)-2-(3-carbamoylphenoxyimino)acetamide]-3-(6,7-dihydroxy-2-isoquinolinium)methyl-3-cephem-4-carboxylate

(15) (6R,7R)-7-[(Z)-2-(2-amino-1,3-thiazol-4-yl)-2-(3-trifluoromethylphenoxyimino)acetamide]-3-(6,7-dihydroxy-2-isoquinolinium)methyl-3-cephem-4-carboxylate

(16) (6R,7R)-7-[(Z)-2-(2-amino-1,3-thiazol-4-yl)-2-(2-methoxyphenoxyimino)acetamide]-3-(6,7-dihydroxy-2-isoquinolinium)methyl-3-cephem-4-carboxylate

(17) (6R,7R)-7-[(Z)-2-(2-amino-1,3-thiazol-4-yl)-2-(4-phenylphenoxyimino)acetamide]-3-(6,7-dihydroxy-2-isoquinolinium)methyl-3-cephem-4-carboxylate

(18) (6R,7R)-7-[(Z)-2-(2-amino-1,3-thiazol-4-yl)-2-(4-carboxymethoxyphenoxyimino)acetamide-3-(6,7-dihydroxy-2-isoquinolinium)methyl-3-cephem-4-carboxylate

(19) (6R,7R)-7-[(Z)-2-(2-amino-1,3-thiazol-4-yl)-2-(4-cyanophenoxyimino)acetamide]-3-(6,7-dihydroxy-2-isoquinolinium)methyl-3-cephem-4-carboxylate

(20) (6R,7R)-7-[(Z)-2-(2-amino-1,3-thiazol-4-yl)-2-(3,4-dichlorophenoxyimino)acetamide]-3-(6,7-dihydroxy-2-isoquinolinium)methyl-3-cephem-4-carboxylate

(21) (6R,7R)-7-[(Z)-2-(5-amino-1,2,4-thiadiazol-3-yl)-2-phenoxyimino)acetamide]-3-(6,7-dihydroxy-2-isoquinolinium)methyl-3-cephem-4-carboxylate

(22) (6R,7R)-7-[(Z)-2-(5-amino-1,2,4-thiadiazol-3-yl)-2-(4-chlorophenoxyimino)acetamide]-3-(6,7-dihydroxy-2-isoquinolinium)methyl-3-cephem-4-carboxylate

(23) (6R,7R)-7-[(Z)-2-(5-amino-1,2,4-thiadiazol-3-yl)-2-(2-chlorophenoxyimino)acetamide]-3-(6,7-dihydroxy-2-isoquinolinium)methyl-3-cephem-4-carboxylate

(24) (6R,7R)-7-[(Z)-2-(2-amino-1,3-thiazol-4-yl)-2-phenoxyiminoacetamide]-3-[4-carboxy-6,7-dihydroxy-2-isoquinolinium)methyl-3-cephem-4-carboxylate

(25) (6R,7R)-7-[(Z)-2-(2-amino-1,3-thiazol-4-yl)-2-phenoxyiminoacetamide]-3-(1-chloro-4-carboxy-6,7-dihydroxy-2-isoquinolinium)methyl-3-cephem-4-carboxylate

(26) (6R,7R)-7-[(Z)-2-(2-amino-1,3-thiazol-4-yl)-2-phenoxyiminoacetamide]-3-(4-cyano-6,7-dihydroxy-2-isoquinolinium)methyl-3-cephem-4-carboxylate

(27) (6R,7R)-7-[(Z)-2-(2-amino-1,3-thiazol-4-yl-2-phenoxyimino)acetamide]-3-(6-hydroxy-7-sulfonyloxy-2-isoquinolinium)methyl-3-cephem-4-carboxylate

(28) (6R,7R)-7-[(Z)-2-(2-amino-1,3-thiazol-4-yl-2-phenoxyimino)acetamide]-3-(6-hydroxy-7-phosphoniumoxy-2-isoquinolinium)methyl-3-cephem-4-carboxylate

(29) (6R,7R)-7-[(Z)-2-(2-amino-1,3-thiazol-4-yl-2-phenoxyimino)acetamide]-3-(6,7-dioxy-di-tert-butylsilyl-2-isoquinolinium)methyl-3-cephem-4-carboxylate

The cephalosporin compound (I), its salt and its ester in this invention can be formed in varied synthesis methods. Several of the synthesis methods are described below. However, the cephalosporin compound of this invention is not limited at all by these synthesis methods.

<u>Reaction scheme A</u>

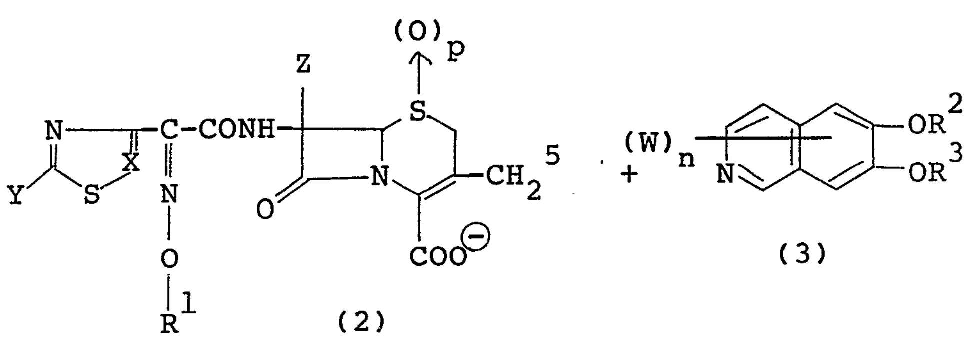

(2)

(3)

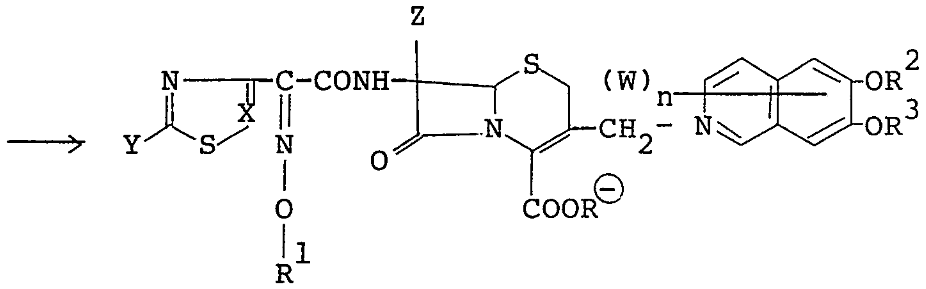

... I

In the reaction scheme A, the cephalosporin compound of formula (2) is reacted with the isoquinoline compound of formula (3). The reaction product may be subjected to deprotection, salt formation, esterification and/or reduction if required.

In formula (2) X, Y, Z and $R^1$ are as defined in formula (1), $R^5$ denotes an acyloxy group, a carbamoyloxy group or a halogen atom, p is 0 or 1 and $R^4$ denotes a hydrogen atom or a protective group.

Preferable examples of the acyloxy group in $R^5$ are acetyloxy, trifluoroacetyloxy, trichloroacetyloxy, propionyloxy, 3-oxobutylyloxy, 3-carboxypropionyloxy, 2-carboxybenzoyloxy, 4-carboxybutylyloxy, mandelyloxy, 2-(carboethoxycarbamoyl)benzoyloxy, 2-(carboethoxy-sulfamoyl)benzoyloxy and 3-ethoxycarbamoylpropionyloxy. Examples of the halogen atom are iodine, bromine and chlorine.

Examples of the protective group in $R^4$ are lower alkyl groups such as methyl, ethyl, propyl, iso-propyl, butyl, pentyl and hexyl; alkanoyloxyalkyl groups such as acetoxymethyl, propionyloxymethyl, butyryloxy-methyl, valeryloxymethyl and pivaloyloxymethyl; alkoxy-alkyl groups such as methoxymethyl, ethoxymethyl, methoxyethyl and ethoxyethyl; aralkyl groups that may be substituted, such as benzyl, 4-methoxybenzyl, 4-nitrobenzyl, phenethyl, trityl, diphenylmethyl, bis(methoxyphenyl)-methyl and 3,4-dimethoxybenzyl; halogenated alkyl groups such as 2-iodoethyl and 2,2,2-trichloroethyl; alkenyl groups such as vinyl and allyl; aryl groups that may be

substituted, such as phenyl, 4-chlorophenyl, tolyl, xylyl and mesityl; and trialkylsilyl groups such as trimethylsilyl, triethylsilyl, tert-butyldimethylsilyl, and tributylsilyl. Above all, the trialkylsilyl groups are preferable as $R^4$. When $R^4$ is the trialkylsilyl groups, formation of a $\triangle^2$-isomer of the cephalosporin derivative represented by formula (1) is suppresed in the reaction scheme A, whereby the intended cephalosporin compound of formula (1) is obtained in high yield.

The cephalosporin compound of formula (2) may be in salt form. Preferable examples of the salt can be the same salts as shown in the cephalosporin derivatives of formula (1).

W and n in formula (3) are as defined in formula (1).

The cephalosporin compound of formula (3) may be in salt form. Preferable examples of the salt are inorganic salts such as a hydrochloride, a hydrobromide, a sulfate and a phosphate; and organic acid salts such as an acetate, a maleate, a tartarate, a benzenesulfonate and a toluenesulfonate.

The reaction between the cephalosporin compound of formula (2) or its salt and the isoquinoline compound of formula (3) or its salt is carried out by contacting them in an inactive organic solvent.

Examples of the inactive organic solvent are halogenated hydrocarbons such as methylene chloride, dichloromethane, chloroform, carbon tetrachloride and 1,2-dichloroethane, ethers such as diethylether, tetrahydrofuran, dioxane and dimethoxyethane, hydrocarbons such as hexane, benzene, toluene and xylene, acetonitrile, dimethylformamide, diethyl acetamide, dimethylsulfoxide, and ethyl acetate.

The reaction temperature is preferably room temperature or lower. It is advisable to perform the reaction usually in the temparature range of -30°C to +50°C.

The cephalosporin compound of formula (2) or its salt and the isoquinoline compound of formula (3) or its salt are reacted in equimolar amounts. In the reaction, the isoquinoline compound of formula (3) or its salt is used in an amount of 0.7 to 10 mols, preferably 1.0 to 10 mols per mol of the cephalosporin compound of formula (2) or its salt.

The reaction proceeds only by stirring both of them. The reaction time varies with reaction solvent and reaction temperature. It is usually 5 minutes to 3 hours. The reaction product can be isolated and purified by known means such as solvent extraction, crystallization and chromatography.

When in the reaction scheme A a syn-isomer of the compound of formula (2) is used as a starting compound, a syn-isomer of the end compound of formula (1) is obtained. When a mixture of syn- and anti-isomers of the compound (2) is used as a starting compound, a mixture of syn- and anti-isomers of the end compound (1) is obtained. The mixture can be separated by usual means such as crystallization and chromatography.

The reaction product is subjected to deprotection, salt formation, esterification and/or reduction if required. The deprotection reaction is a reaction known per se, and the protective group of the carboxyl group can be removed by a hydrolysis reaction in the presence of an acid or an alkali. The protective group of the amino group can be removed by acid or catalytic reduction (refer to U.S. Patent Nos. 4,152,433 and 4,298,606).

The salt formation reaction is a reaction known per se. For example, it is carried out by treating the compound of a betaine structure of formula (1) obtained by the above reaction with an acid such as hydrochloric acid, hydrobromic acid, sulfuric acid, phosphoric acid, acetic acid or trifluoroacetic acid or with a salt such as sodium chloride, sodium iodide or potassium chloride

in the presence of an acid.

The esterification reaction is a reaction known per se, and it is carried out by reacting the resulting cephalosporin compound of formula (1) or its salt with an alkanoyloxyalkyl halide or an alkoxyalkyl halide in an inactive organic solvent such as acetone or dimethylformamide (refer to British Patent No. 240921).

The reduction reaction is a reaction known per se, and it is carried out by treating the cephalosporin compound of formula (1) wherein p is 1 with acetyl chloride, and reducing the treated compound with an iodine ion or sodium dithionite (refer to British Patent No. 240921).

The cephalosporin compound of this invention in crystalline form is obtained by the following methods.

A method wherein the amorphous powder of the cephalosporin compound of formula (1) is dissolved in a soluble organic solvent or a mixture of the soluble organic solvents, left to stand and precipitated as a crystal; a method wherein the amorphous powder is recrystallized with the above solvent or solvent mixture; and a method wherein the amorphous powder is dissolved in water, and then precipitated as a crystal with the addition of the above solvent or solvent mixture.

There is another method wherein an acid addition salt of the cephalosporin compound of formula (1) is dissolved in an inactive, soluble water or organic solvent or a solvent mixture of these and then neutralized with a base to obtain a crystal.

When crystallization or recrystallization is carried out, as aforesaid, in water or an aqueous solution, crystalline hydrates are usually obtained. When crystallization is carried out in an organic solvent, crystalline anhydrides or solvates are obtained. The solvates are easily converted into hydrates. The crystalline anhydrides or solvates can be converted into hydrates by allowing them to stand in a steam-containing gas.

Reaction scheme B

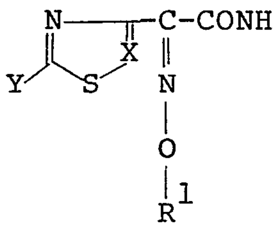 + 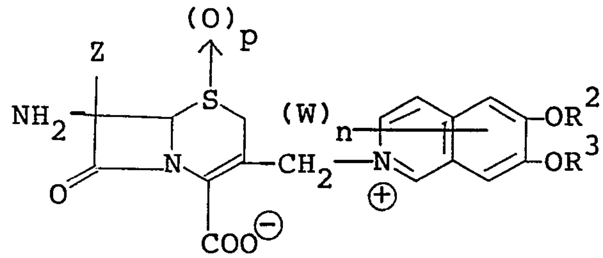

(4) (5)

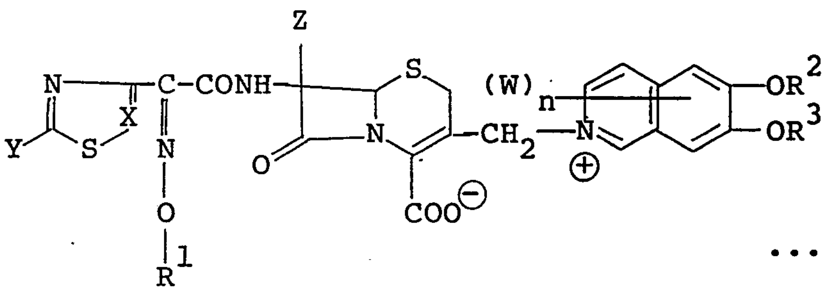

... (I)

In the reaction scheme B, the compound of formula (4), its salt or its reactive derivative is reacted with the compound of formula (5), its salt, its ester or its reactive derivative. The reaction product is subjected to deprotection, salt formation, esterification and/or reduction if required.

In formula (4), X, Y and $R^1$ are as defined in formula (1).

The compound of formula (4) may be in salt form, and the salt is an acid addition salt. Examples of such acid addition salt can be the same as the acid addition salts of the compound of formula (3) in the reaction scheme A.

Examples of the reactive derivative of the compound of formula (4) are an acid halide, an acid anhydride, a mixed acid anhydride activated amide and an activated ester.

Examples of the acid halide are an acid chloride and an acid bromide. Examples of the mixed acid anhydride are anhydrides of mixtures of acids such as a dialkylphosphoric acid, phenylphosphoric acid, pivalic acid and pentanoic acid. Examples of the activated amide are amides activated with imid azole, triazole, tetrazole and dimethylpyrazole. Examples of the activated ester are cyanomethyl ester, methoxymethyl ester, dimethyliminomethyl ester, p-nitrophenyl ester and mesylphenyl ester.

In formula (5), Z, $R^2$, $R^3$, W and n are as defined in formula (1). Examples of the salt of the compound of formula (5) are alkali metal salts such as a sodium salt and a potassium salt; alkaline earth metal salts such as a calcium salt, and a magnesium salt; an ammonium salt; salts with organic bases such as a trimethylammonium salt, a triethylammonium salt, a pyridinium salt, a tetraethylammonium salt and a tetra-n-butylammonium salt; and the same acid addition salts as snown above.

Examples of the ester of the compound of formula (5) can be compounds esterified with the protective group in $R^2$ of the compound of formula (2) in the reaction scheme A.

Examples of the reactive derivative of the compound of formula (5) are Schiff base-type imino compounds formed by the reaction with a carbonyl compound such as acetoacetic acid or their enamine-type isomers; silyl derivatives formed by the reaction with silyl compounds such as bis(trimethylsilyl)acetamide, trimethylchlorosilane and tert-butyldimethylchlorosilane; and derivatives obtained by the reaction with phosphorus trichloride and phosgene.

The compound of formula (5) can be produced by the following method.

Synthesis method of the compound of formula (5)

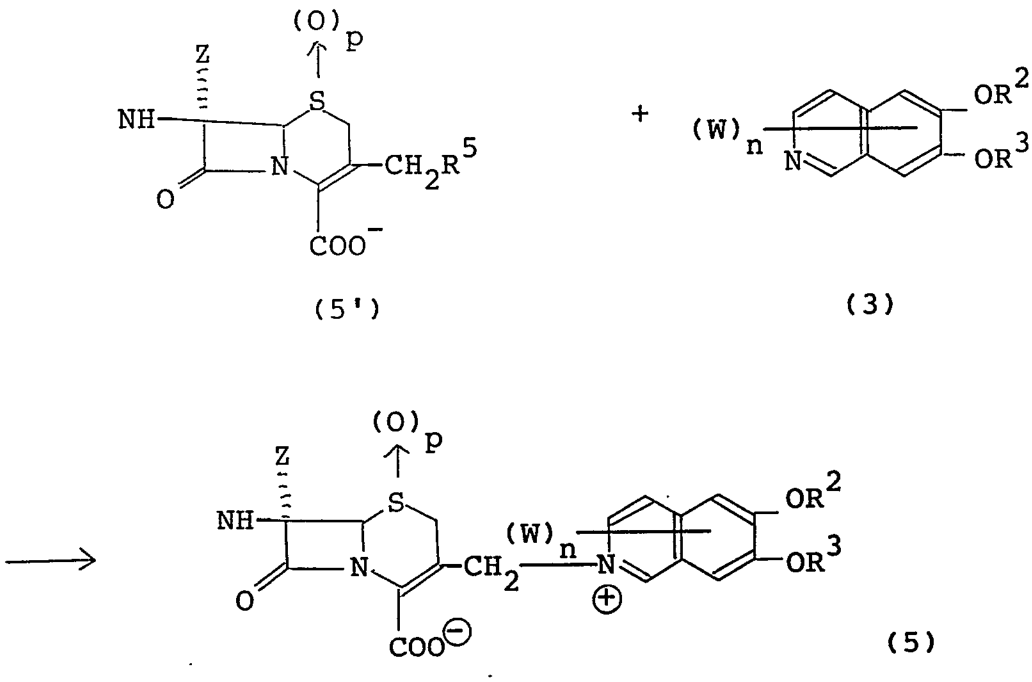

(5') (3)

(5)

The above reaction can be carried out in the same way as the reaction between the compound of formula (2) and the isoquinoline compound of formula (3) by the reaction scheme A.

The reaction of the compound of formula (4), its salt or its reactive derivative with the compound of formula (5), its salt, its ester or its reactive derivative is performed by contacting both of them in an inactive organic solvent and if required, in the presence of a condensation agent or a base.

Examples of the inactive organic solvent are halogenated hydrocarbons such as methylene chloride, dichloromethane, chloroform, carbon tetrachloride and 1,2-dichloroethane, ethers such as diethyl ether, tetrahydrofuran, dioxane and dimethoxyethane, hydrocarbons such as hexane, benzene, toluene and xylene, acetonitrile, dimethylformamide and diethyl sulfoxide.

In the reaction, a base may be added. Examples of the base are inorganic bases such as an alkali metal

hydroxide, an alkali metal bicarbonate and an alkali metal carbonate; and organic bases such as a trialkylamine, a N,N-dialkylbenzylamine, pyridine and N-alkylmorpholine. The amount of such base is usually 1 to 3 mols per mol of the compound of formula (4), its salt, or its reactive derivative.

When the compound of formula (4) or its salt is used in the reaction, the condensation agent may be added. Examples of the condensation agent are N,N'-dicyclohexylcarbodiimide, N-cyclohexyl-N'-morpholinoethylcarbodiimide, N,N'-diethylcarbodiimide, trimethylphosphite, triphenylphosphine, and 2-ethyl-7-hydroxybenzisoxazolium salt. The amount of such condensation agent is 1 to 3 mols per mol of the compound of formula (4) or its salt.

The compound of formula (4), its salt or its reactive derivative and the compound of formula (5), its salt or its reactive derivative are reacted usually in approximately equimolar amounts.

The reaction is usually performed under cooling or at room temperature. The reaction is usually finished in several minutes to several tens of hours. The reaction product can be isolated and purified by known means such as solvent extraction, crystallization and chromatography.

The deprotection, salt formation, esterification and/ or reduction of the reaction product can be carried out in the same way as shown in the reaction scheme A. Even the crystal of the end compound can be afforded in the same manner as shown in the reaction scheme A.

When a syn-isomer of the compound of formula (4) is used as a starting compound in this reaction, a syn-isomer of the end compound of formula (1) is obtained. When a mixture of syn- and anti-isomers of the compound of formula (4) is used as a starting compound, a

mixture of syn- and anti-isomers of the end compound of formula (1) is obtained. Such mixture can be separated by usual means such as crystallization and chromatography.

Reaction scheme C

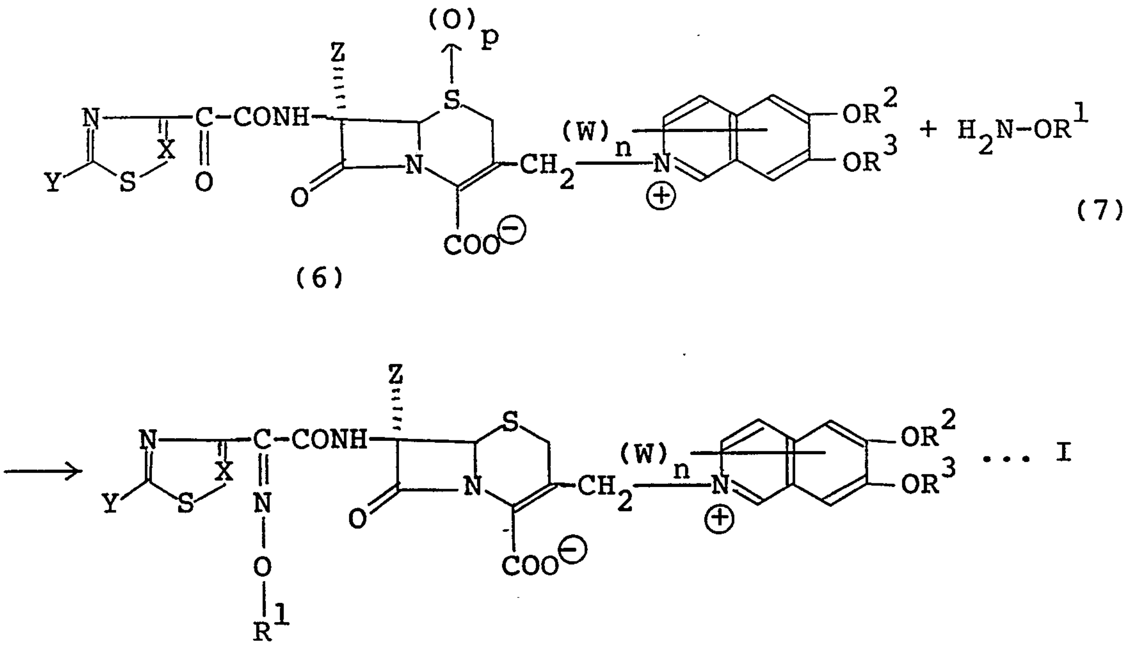

(6)

(7)

... I

In the reaction scheme C, the compound of formula (6), its salt or its ester is reacted with the compound of formula (7), its protected compound or its salt. The reaction product is subjected to deprotection, salt formation, esterification and/or reduction if required.

In formula (6), X, Y, Z, $R^2$, $R^3$, W, n and p are as defined above.

Examples of the salt of the compound of formula (6) are an acid addition salt, an alkali metal salt, an alkaline earth metal salt, an ammonium salt and a salt with an organic base. Concrete Examples of these salts can be the same salts of the compound of formula (1).

The ester of the compound of formula (6) can be, for example, a compound esterified with the group shown as the protective group in $R^4$ of the compound of formula (2) in the reaction scheme A.

The compound of formula (6) can be formed by the following method.

Synthesis method of the compound of formula (6)

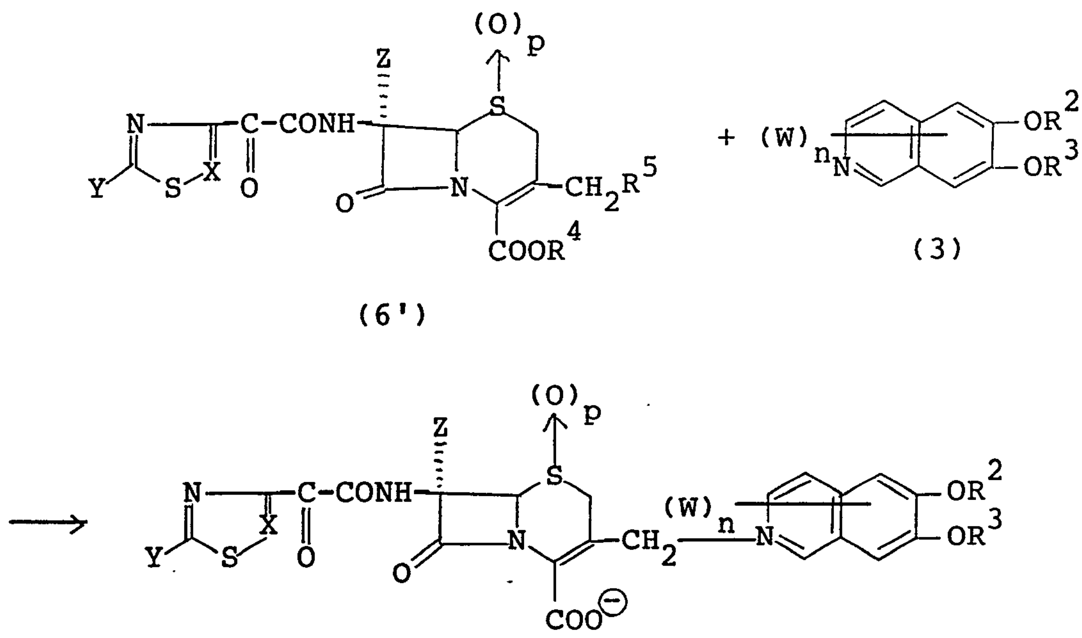

(6')

(3)

(6)

The above reaction can be performed in the same way as the reaction between the compound of formula (2) and the isoquinoline compound of formula (3) by the reaction scheme A.

In formula (7), $R^1$ is as defined above. Regarding the protected one of the compound of formula (7), when $R^1$ has an active substituent such as a carboxyl group, a compound with the substituent protected by an ordinary protective group can be taken.

The salt of the compound of formula (7) is an acid addition salt and concrete examples thereof can be the same as indicated above.

The compound of formula (7) is a known compound and can be formed by a known method using hydroxyphthalimide (Bulletin Society, 833, 1976) or a method using 3,4-dinitrophenolhydroxyamine (Japanese Laid-open Patent Application No. 169446/ 1985).

The reaction of the compound of formula (6), its salt or its ester with the compound of formula (7), its protected compound or its salt is carried out by contacting them in an inactive organic solvent and if required, in the presence of a base.

Examples of the inactive organic solvent are alcohols such as methanol, ethanol, propanol and butanol. These inactive organic solvents may contain water.

Examples of the base which is added if required can be the same as the examples of the base used in the reaction by the reaction scheme B.

The amount of such base is usually 1 to 3 mols per mol of the compound of formula (6), its salt or its ester. The compound of formula (6), its salt or its ester and the compound of formula (6), its protected compound or its salt are usually reacted in equimolar amounts.

The reaction is usually carried out under cooling or at room tempersture. The reaction time is usually several minutes to several tens of hours. The reaction product can be isolated and purified by known means such as solvent extraction, crystallization and chromatography. In this reaction, the mixture of the syn- and anti-isomers of the compound is obtained.

Such mixture can be separated by usual means such as crystallization and chromatography.

Deprotection, salt formation, esterification and/or reduction can be conducted in the same way as in the reaction scheme A. The crystalline anhydride or hydrate of the end compound can be also btained in the same way as in the reaction scheme A.

Reaction scheme D

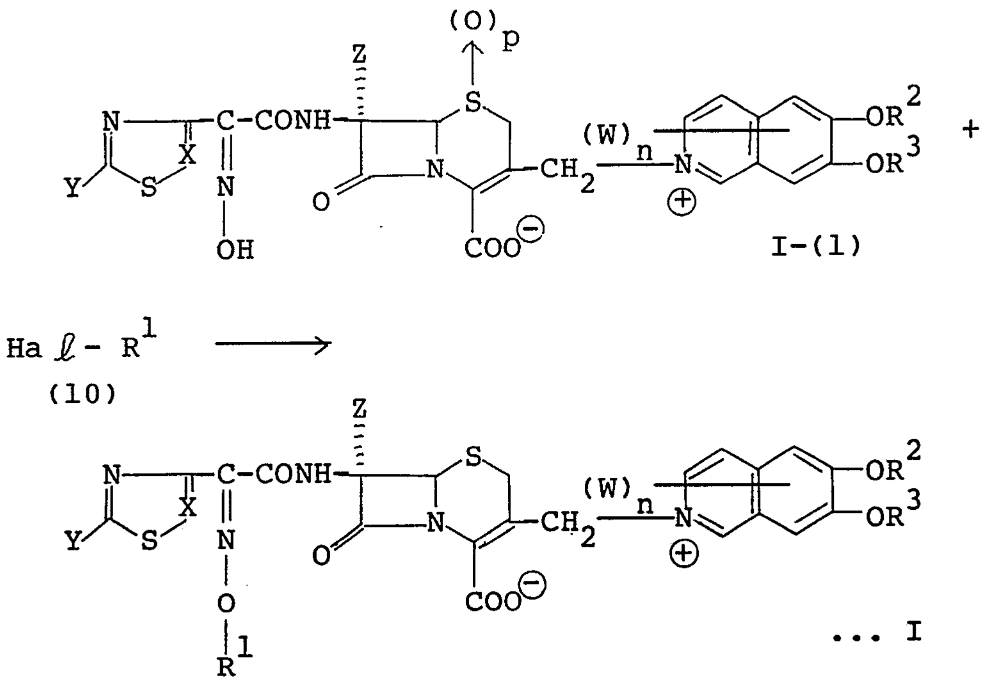


In the reaction scheme D, the compound of formula I-(1), its salt or its ester is reacted with the compound of formula (10) in the presence of a dialkyl sulfate or a diazoalkane. The reaction product is subjected to deprotection, salt formation, esterification and/or reduction.

In formula I-(1), X, Y, Z, $R^1$, $R^2$, W, n and p are as defined above.

Examples of the salt of the compound of formula I-(1) are an acid addition salt, an alkali metal salt, an alkaline earth metal salt, an ammonium salt and a salt with an organic base. Concrete examples of these salts can be the same salts shown in the compound of formula (1).

The ester of the compound of formula I-(1) can be a compound esterified with the protective group in $R^4$ of the compound of formula (2) in the reaction scheme A.

The compound of formula I-(1) can be formed by the following method.

Synthetic method of the compound of formula I-(1)

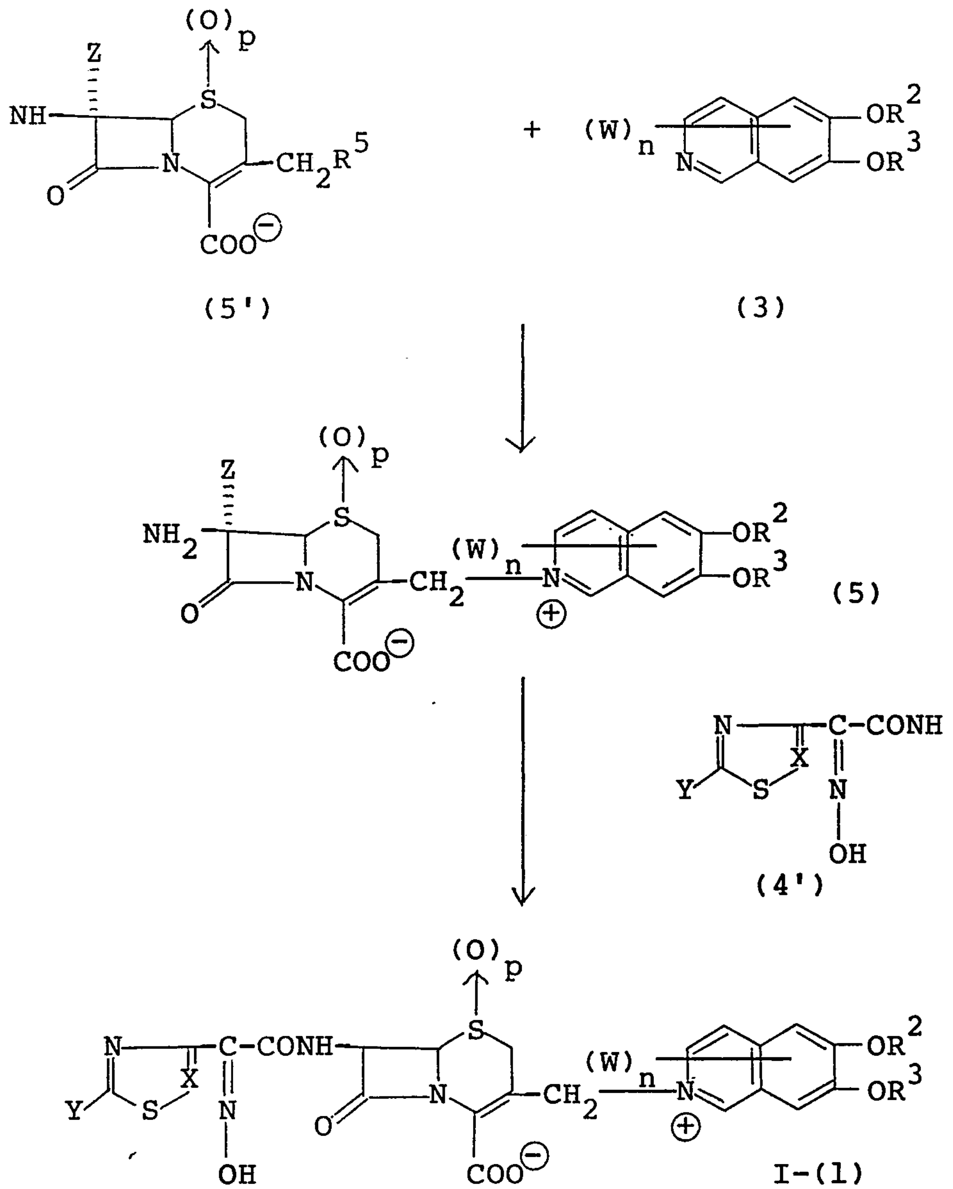

(5') (3) (5) (4') I-(1)

In the above method, the reaction of the compound of formula (5') with the compound of formula (3) can be carried out in the same way as the reaction by the reaction scheme A, and the reaction of the compound of formula (5) with the compound of formula (4') in the same way as the reaction by the reaction scheme B respectively. The compound of formula (4') is a known compound which can be formed by a known method (U.S. Patent No. 4,298,606).

In formula (10), Hal denotes a halogen atom such as chlorine, bromine or iodine. The compound of formula (10) is obtained by halogenating the corresponding aromatic hydrocarbon by a known means.

Examples of the dialkyl sulfate are dimethyl sulfate and diethyl sulfate.

Examples of the diazoalkane are diazomethane, etc.

The reaction of the compound of formula I-(1), its salt or its ester with the compound of formula (10) in the presence of a dialkyl sulfate or a diazoalkane is carried out by contacting them in an inactive solvent and if required, in the presence of a base.

Examples of the inactive solvent are alcohols such as methanol, ethanol, propanol and butanol, halogenated hydrocarbons such as methylene chloride, dichloromethane, chloroform, carbon tetrachloride and 1,2-dichloroethane, ethers such as diethyl ether, tetrahydrofuran, dioxane and dimethoxyethane, dimethylformamide, diethyl acetamide, ethyl acetate and water.

If required, the reaction may be run in the presence of a base. Examples of such base are inorganic bases such as an alkali metal hydroxide, an alkali metal bicarbonate and an alkali metal carbonate; and organic bases such as a trialkylamine, a N,N-dialkylbenzylamine, pyridine and N-alkylmorpholine. The amount of such base is usually 1 to 3 mols per mol of the compound of formula I-(1), its salt or its ester.

The amount of the compound of formula (10), the dialkyl sulfate or the diazoalkane is usually 1 to 3 mols per mol of the compound of formula I-(1), its salt or its ester.

The reaction temperature is not limited in particular; the reaction is usually conducted under cooling or under acceleration at a boiling point of a solvent.

The reaction time is usually several minutes to several tens of hours.

The reaction product can be isolated and purified by known means such as solvent extraction, crystallization and chromatography. When a syn-isomer of the compound of formula I-(1) is used as a starting compound in this reaction, a syn-isomer of the compound of formula I-(1) is obtained. When a mixture of the syn- and anti-isomers of the compound of formula I-(1) is used as a starting compound, a mixture of the syn- and anti-isomers of the compound I-(1) is obtained. Such mixture can be isolated by crystallization and chromatography.

Deprotection, salt formation, esterification and reduction can be carried out in the same way as in the method by the reaction scheme A. The crystalline anhydride or hydrate of the end compound can be obtained also in the same way as shown in the method by the reaction scheme A.

<u>Reaction scheme E</u>

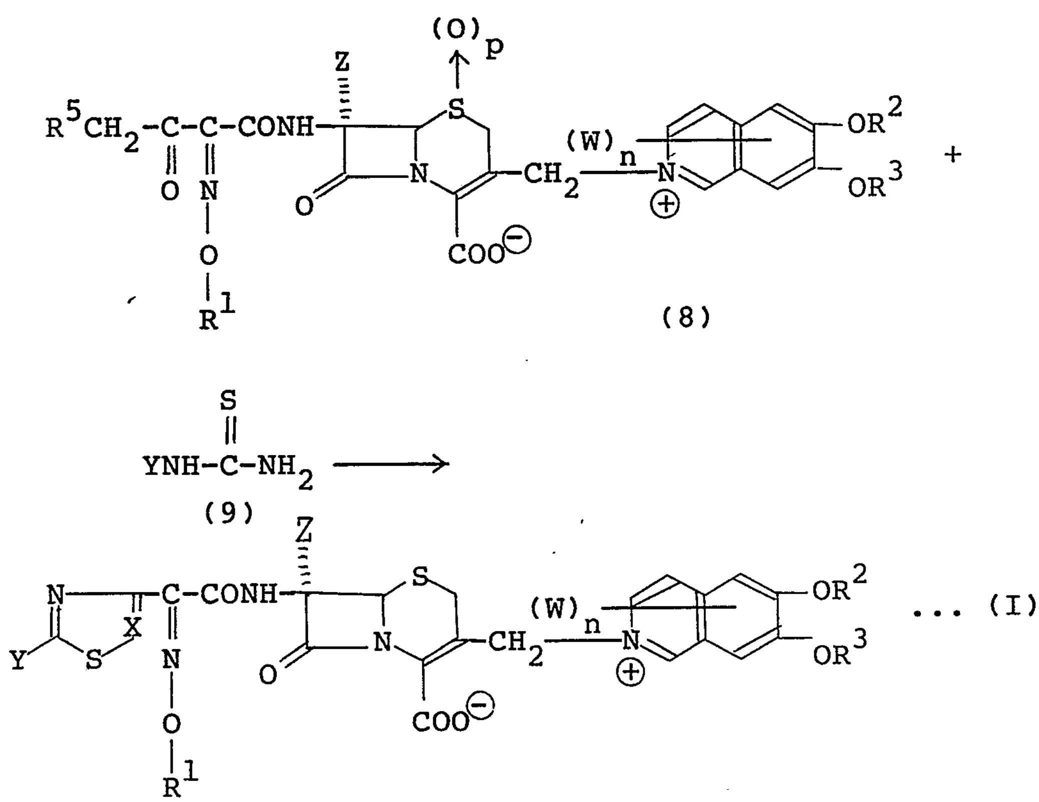

(8)

(9)

... (I)

The reaction scheme E is a synthetic method of the compound of formula I wherein X is -CH=.

In the reaction scheme E, the compound of formula (8), its salt or its ester is reacted with the compound of formula (9) or its salt. The reaction product is subjected to deprotection, salt formation, esterification and/or reduction if required.

In formula (8), Y, Z, $R^1$, $R^2$, $R^3$, W, n and p are as defined above, and $R^6$ denotes a halogen atom such as chlorine, bromine or iodine.

Examples of the salt of compound of formula (8) are an alkali metal salt, an alkaline earth metal, an ammonium salt, a salt with an organic base and an acid addition salt. Concrete examples of these salts can be the same salts as indicated in the compound of formula (1).

The ester of the compound of formula (8) can be a compound esterified with the protective group in $R^4$ of the compound of formula (2) in the reaction scheme A.

The compound of formula (8) is formed by the following method.

Synthetic method of the compound of formula (8)

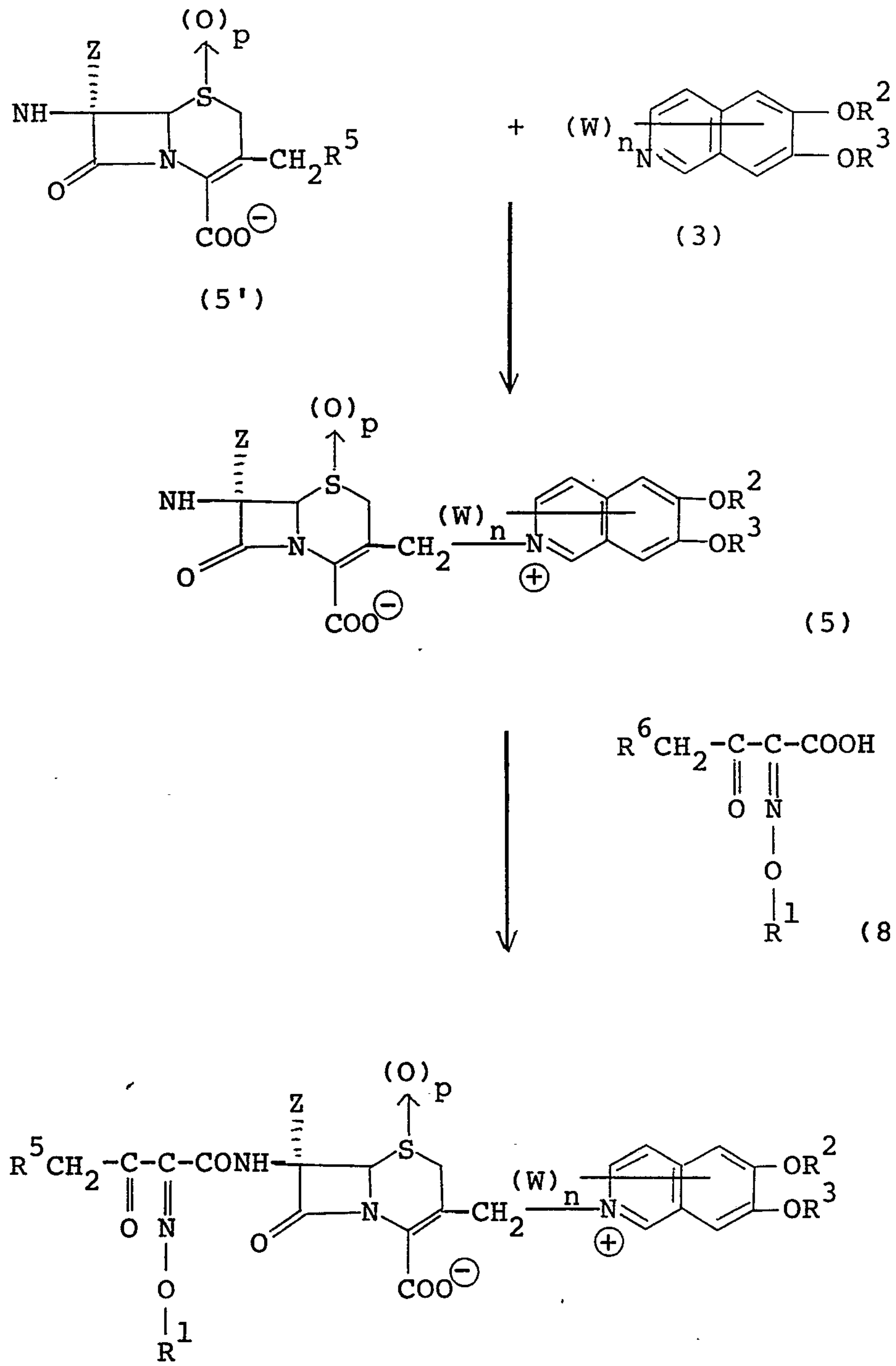

(5')

(3)

(5)

(8')

(8)

In the above method, the reaction of the compound of formula (5') with the compound of formula (3) is carried out in the same way as the reaction by the reaction scheme A, and the reaction of the compound of formula (5) with the compound of formula (8') in the same way as the reaction by the reaction scheme B respectively. The compound of formula (8') is a known compound which can be formed by a known method (e.g. British Patent No. 2,012,276).

In formula (9), $R^1$ denotes a hydrogen atom or a protective group. The compound of formula (9) is a known compound (U.S. Patent No. 4,298,606).

The salt of the compound of formula (9) is an acid addition salt, and concrete examples of such acid addition salt can be the same acid addition salts described in the compound of formula (1).

The reaction of the compound of formula (8), its salt or its ester with the compound of formula (9) or its salt can be performed by contacting them in an inactive solvent.

Examples of the inactive solvent are water, alcohols such as methanol, ethanol, propanol and butanol, ethers such as diethyl ether, tetrahydrofuran and dioxane, dimethylformamide, dimethylacetamide and methylpiperidone. A mixture of these solvents is also available.

The amount of the compound of formula (9) or its salt is usually 1 to 3 mols per mol of the compound of formula (8), its salt or its ester.

The reaction temperature is not limited in particular; the reaction is usually carried out at a temperature of from room tempearture to a boiling point of a solvent.

The reaction time is usually about 1 to 10 hours.

The reaction product can be isolated and purified by known means such as solvent extraction, crystallization and chromatography.

The deprotection, acid formation, esterification and/or reduction can be carried out in the same way as in the method by the reaction scheme A. The crystalline anhydride or hydrate of the end product can be obtained in the same way as in the method by the reaction scheme A.

In this invention, the cephalosporin compound of formula (1), above all, the cephalosporin compound of formula (1) with Y of an amino group, its pharmaceutically acceptable salt or its ester shows high antibacterial activity to wide-ranging Gram-positives and Gram-negatives and is quite useful in therapy of microbism of animals including humans. The cephalosporin compound in this invention has high antibacterial activity to Gram-negatives such as Pseudomonas aeruginosa, Salmonella typhi and Enterobacter, and Gram-positives such as Staphylococcus aureus, Streptococcus pyogenes and Bacillus subtilis. The cephalosporin derivative in this invention possesses the characteristic that it has a long duration time.

The cephalosporin compound, its pharmaceutically acceptable salt or its ester in this invention is administered either parenterally or orally. Dosage forms in case of the parenteral administration are, for example, an intravenous or intramuscular injection and a suppository. The injection can take a form of an aqueous solution, an active solution or a suspension. A powder and crystals used by treatment with sterilized water before administration are also available. The suppository can be a preparation obtained by containing an absorption accelerator, if required, in an ordinary excipient such as cocoa butter or glyceride.

In case of the oral administration, there are ordinary capsules, tablets and granules; they may contain an absorption accelerator and a preservative.

These various preparations can be formed by a method known per se.

The dose of the cephalosporin compound, its pharmaceutically acceptable salt or its ester varies with age and conditions of patients, and dosage forms; it is usually 10 to 2000 mg/day.

The following Examples illustrate this invention in more detail.

Example 1: (6R,7R)-7-[(Z)-2-(2-amino-1,3-thiazol-4-yl)-2-phenoxy iminoacetamide]-3-(6,7-dihydroxy-2-isoquinolinium) methyl-3-cephem-4-carboxylate

605 mg of (6R,7R)-7-[2-(2-chloroacetamide-1,3-thiazol-4-yl)-2-oxoacetamide]-3-(6,7-dihydroxyisoquinolinium)methyl-3-cephem-4-carboxylate was dissolved in 5 ml of methanol and 1.0 ml of N,N'-dimethylformamide. 109 mg of o-phenylhydroxylamine was added, and the reaction was run at room temperature for 16 hours. After the reaction, the solvent was distilled off under reduced pressure, 100 mg of thiourea was then added and the mixture was stirred at room temperature for 3 hours.

Subsequently, the reaction solution was added to 100 ml of acetone to obtain a precipitate. The resulting compound was then collected by liquid chromatography [column: RP-18, elution solvent: water/methanol solvent mixture, detection: UV wavelength 260 nm] to obtgain an end compound (90 mg).

IR ($cm^{-1}$) : 1770

NMR ($d_6$ - DMSO - $D_2O$) $\delta$:

5.12 1H (d)

5,70 1H (d)

6.60 1H (s)

6.89-7.45 PH (m)

8.67 1H (s)

Example 2: (6R,7R)-7-[(Z)-2-(2-amino-1,3-thiazol-4-yl)-2-phenoxyiminoacetamide]-3-(6,7-dihydroxy-2-isoquinolinium)methyl-3-cephem-4-carboxylate

(i) In 2 ml of acetonitrile was suspended 200 mg of (6R,7R)-7-amino-3-iodomethyl-3-cephem-4-carboxylic acid. While cooling on an ice bath, 435 microliters of bistrimethylsilylacetamide was added dropwise. After the addition, stirring was conducted for 1 hour under ice cooling. The mixture was then cooled to -20°C to obtain trimethylsilyl (6R,7R)-7-bistrimethylsilylamino-3-iodomethyl-3-cephem-4-carboxylate. To this was added dropwise slowly a solution of 74 g of 6,7-dihydroxy-isoquinoline in 400 microliters of N,N'-dimethylformamide with stirring. There resulted an acetonitrile solution of trimethylsilyl (6R,7R)-7-bistrimethylsilylamino-3-(6,7-dihydroxy-2-isoquinolinium)methyl-3-cephem-4-carboxylate.

(ii) The temperature of the resulting solution was raised to room temperature.

A solution of (Z)-2-phenoxyimino-2-(2-trityl-amino-1,3-thiazol-4-yl)acetic acid chloride prepared separately from 300 mg of (Z)-2-phenoxyimino-2-(2-trityl-amino-1,3-thiazol-4-yl)acetic acid and 132 mg of phosphorus pentachloride in 1 ml of dichloromethane was added to the above solution at a time. After stirring the solution at room temperature for 1 hour, most of the solution was removed under reduced pressure, and 600 microliters of trifluoroacetic acid containing 10% of water was added, followed by stirring at room temperature for 2 hours. Most of trifluoroacetic acid was distilled off under reduced pressure, and with vigorous stirring, 5 ml of diethyl ether was added. The resulting precipitate was separated by filtration, and extracted with about 1 milliliter of a methanol/water solvent mixture (MeOH 8: water 2). After the extract was concentrated, about 2 ml of ethanol was added, and the concentrate was left to

cool. When the resulting precipitate was filtered, there resulted trifluoroacetate of (6R,7R)-7-[7-(Z)-2-(2-amino-1,3-thiazol-4-yl)phenoxyiminoacetamide]-3-(6,7-dihydroxy-2-isoquilinium)methyl-3-cephem-4-carboxylate.

Said trifluoroacetate was dissolved in water, and the solution was passed through a Na-type Amberlite IRA-410 ion exchange resin column and lyophilized. Purification was conducted in a water/methanol solvent mixture using a HP-20 ion exchange resin column while gradually increasing a methanol volume ratio from 0% to 40%.

A fraction eluted with 20% to 40% of methanol was lyophilized to obtain final (6R,7R)-7-[(Z)-2-phenoxy-2-(2-aminothiazol-4-yl)acetamide]-3-(6,7-dihydroxy-2-isoquinolinium)methyl]-3-cephem-4-carboxylate. The spectrum data (IR, NMR) agreed with those obtained in Example 1.

Example 3: (6R,7R)-7-[(Z)-2-(5-amino-1,2,4-thiadiazol-3-yl-2-[phenoxyiminoacetamide]-3-(6,7-dihydroxy-2-isoquinolinium)methyl-3-cephem-4-carboxylate

In 5 ml of acetonitrile was suspended 340 mg of (6R,7R)-7-amino-3-iodomethyl-3-cephem-4-carboxylic acid. While cooling on an ice bath, 0.74 ml of BSA(bistrimethylsilylacetamide) was added dropwise, and the mixture was stirred for 1 hour under ice cooling. At the same temperature, a solution of 300 mg of 6,7-di-tert-butylsiloxyisoquinoline in 2 ml of acetonitrile was added, and the mixture was stirred for 30 minutes.

Meanwhile, an acid chloride solution prepared from 510 ml of (Z)-2-phenoxyimino-2-(2-tritylamino-1,3-thiazol-4-yl)acetic acid and 220 mg of phosphorus pentachloride in 5 ml of dichloromethane was added at a time, and the temperature was slowly raised to room temperature, followed by stirring for 1 hour. After the reaction was over, 1 ml of methanol was added, and most

of the low-boiling solvents (dichloromethane and acetonitrile) were immediately removed under reduced pressure. The residue was extracted with ethyl acetate, and the extract was water-washed and dried with magnesium sulfate. After ethyl acetate was distilled off, the residue was dissolved in 10 ml of a solution of $CF_3COOH:H_2O=1:1$, and they were vigorously stirred at room temperature for 4 hours. The reaction product was poured in 150 ml of diethyl ether. The obtained precipitate was dissolved again in trifluoroacetic acid and eluted by a CHP-20 resin column using a 0.1% trifuoroacetic acid solution and methanol as an elution solvent by gradually changing a ratio of 0.1% trifuoroacetic acid solution: methanol from 20:80 to 80:20. A fraction eluted at a ratio of trifluoroacetic acid solution:methanol = about 50:50 was collected, concentrated and lyophilized to obtain 80 mg of the captioned compound as trifluoroacetate. The NMR spectrum data of the resulting compound agreed with those obtained in Example 1.

Example 4: (6R,7R)-7-[(Z)-2-(2-amino-1,3-thiazol-4-yl)-2-(p-chlorophenoxy)iminoacetamide]-3-(6,7-dihydroxy-2-isoquinolinium)methyl-3-cephem-4-carboxylate

(6R,7R)-7-[(Z)-2-(2-amino-1,3-thiazol-4-yl)-2-(p-chlorophenoxyimino)acetamide]-3-(6,7-dihydroxy-2-isoquinolinium)methyl-3-cephem-4-carboxy late was obtained in the same way as in Example 1 except using 127 mg of o-(p-chlorophenyl)hydroxylamine instead of o-phenylhydroxylamine.

IR ($cm^{-1}$) : 17

NMR ($d_6$ DMSO - $D_2$ O) $\delta$:

5.20 1H (d)

5.86 1H (d)

6.92-7.83 8H (m)

8.23 2H (d)

9.40 1H (s)

Example 5: (6R,7R)-7-[(Z)-2-(5-amino-1,2,4-thiadiazol-3-yl)-2-phenoxyiminoacetamide]-3-(6,7-dihydroxy-2-isoquinolinium)methyl-3-cephem-4-carboxylate

0.743 g of 7-amino-3-(6,7-dihydroxy-2-isoquinolinium)methyl-3-cephem-4-carboxylate-dihydrochloride was dissolved in 8.3 ml of N,N'-dimethylacetamide, and 0.668 g of triethylamine was added at -20°C. On the other hand, 0.38 g of 5-amino-1,2,4-thiadiazol-2-phenoxyiminoacetic acid was reacted with 0.50 g of $PCl_5$ in 8 ml of dichloromethane at -10°C for 15 minutes. The solution was added to the above N,N'-dimethylacetamide solution at -20°C, and the temperature was progressively elevated to room temperature, followed by stirring for 1 hour. The reaction mixture was then poured in diethyl ether to collect the precipitate by filtration. About 0.9 g of a crude product was obtained. It was dissolved in methanol and eluted via a CPH-20 resin column by changing a ratio of methanol:water from 20:80 to 100:0. From about 20 mg of a fraction eluted at a ratio of methanol: water = 70:30, final (6R,7R)-7-[(Z)-2-(5-amino-1,2,4-thiadiazol-3-yl-2-phenoxyimino)acetamide]-3-(6,7-dihydroxy-2-isoquinolinium)methyl-3-cephem-4-carboxylate (compound XI) was obtained.

IR ($cm^{-1}$) : 1770
NMR ($d_6$ - DMSO - $D_2$ O) $\delta$:
3.5 2H (b)
5.12 1H (d)
5.5 2H (b)
5.90 1H (d)
6.9-7.6 7H (m)
8.1-8.4 2H (d + d)
9.5 1H (s)

Example 6: (6R,7R)-7-[(Z)-2-(2-amino-1,3-thiazol-4-yl-2-phenoxyimino)acetamide]-3-(6,7-hydroxy-7-sulfonyloxy-2-isoquinolinium)methyl-3-cephem-4-carboxylate

1.0 g of (6R,7R)-7-[(Z)-2-(2-tritylamino-1,3-thiazol-4-yl-2-phenoxyimino)acetamide]-3-(6,7-dihydroxy-2-isoquinoliniummethyl)-3-cephem-4-carboxylate was dis solved in 10 ml of DMF, and chlorosulfonic acid was added at room temperature, followed by stirring for 30 minutes. The reaction solution was poured in 150 ml of diethyl ether to collect the precipitate by filtration. About 0.8 g of the obtained precipitate was dissolved in 10 ml of a solvent mixture of trifluoroacetic acid:water = 10:1, and stirred at room temperature for 5 minutes. The mixture was poured into 150 ml of diethyl ether to collect the precipitate by filtration. The precipitate was extracted with water, and the aqueous phase was subjected to reversed phase chromatography by a CHP-20 resin column using a water/methanol solvent mixture. A fraction eluted at a water:methanol = about 8:2 was concentrated and lyophilized to obtain 13.5 mg of the captioned compound.

2 mg of the captioned compound was dissolved in 1 ml of a glycine buffer having pH of 7.0, and 20U of commercial alkali phosphatase (derived from calf intestines) was charged. The solution was kept at 37°C for 1 hour, and then analyzed by liquid chromatography [column: RP-18, elution solvent: a water/methanol solvent mixture, detection: UV wavelength 260 nm]. Comparison was made with respect to the Rf value of the main peak, and it was confirmed to agree with that of (6R,7R)-7-[(Z)-2-(2-amino-1,3-thiazol-4-yl)-2-phenoxyiminoacetamide]-3-(6,7-dihydroxy-2-isoquinoliniummethyl)-3-cephem-4-carboxylate.

Example 7: (6R,7R)-7-[(Z)-2-(2-amino-1,3-thiazol-4-yl-2-phenoxyimino)acetamide]-3-(6-hydroxy-7-phosphonyloxy-2-isoquinolinium)methyl-3-cephem-4-carboxylate

1.0 g of (6R,7R)-7-[(Z)-2-(2-tritylamino-1,3-thiazol-4-yl-2-phenoxyimino)acetamide-3-(6,7-dihydroxy-2-isoquinoliniummethyl)-3-cephem-4-carboxylate was dissolved in 10 ml of DMF, and 0.7 g of pyridine was added. Subsequently, 1.2 g of phosphorus oxychloride was added at 0°C, and the mixture was stirred at the same temperature and then poured in 200 ml of diethyl ether. The precipitate was collected by filtration, stirred in 10 ml of 90% formic acid at room temperature for 30 minutes and poured in 150 ml of diethyl ether. The precipitate was then collected by filtration. Said precipitate was suspended in 20 ml of water and extracted. The aqueous layer was subjected to reversed phase chromatography in a water/methanol solvent mixture using a CHP-20 resin column. A fraction eluted at a ratio of water:methanol = 9:1 to 8:2 was collected and lyophilized to afford 24 mg of the captioned compound.

Example 8: In vitro antibacterial activity

An in vitro antibacterial activity was measured by an agar disc dilution method.

Varied test bacteria were cultivated overnight in triptophane-soybroth. A Mueller Hinton agar containing the compound of this invention in varied concentrations was streaked with one platinum loopful of each of the bacteria (number of living cells $10^6$/ml). Cultivation was conducted at 37°C for 16 hours, and minimum growth inhibitory concentration (MIC, μg/ml) was found. The results are shown in Table I.

Table 1

| | Compound | | | | | | | | | | | | |
|---|---|---|---|---|---|---|---|---|---|---|---|---|---|
| | I | II | III | IV | V | VI | VII | VIII | IX | X | XI | A | CAZ |
| S. aureus Terajima | 0.8 | 0.2 | 0.8 | 1.6 | 1.6 | 3.1 | 3.1 | 3.1 | 1.6 | 1.6 | 0.2 | 6.2 | 3.1 |
| S. pyogenes Cook | 0.2 | 0.2 | 0.1 | 0.4 | 0.8 | 0.8 | 0.4 | 0.8 | 0.4 | 0.8 | 0.05 | 1.6 | 1.6 |
| E. coli K12C600 | <0.01 | <0.01 | <0.01 | 0.02 | 0.02 | 0.02 | 0.03 | 0.05 | 0.01 | <0.01 | 0.01 | <0.01 | 0.2 |
| K. pneumoniae PC I 602 | <0.01 | <0.01 | <0.01 | 0.02 | 0.03 | 0.02 | 0.03 | 0.03 | <0.01 | <0.01 | <0.01 | <0.01 | 0.1 |
| S. typhic 901 | <0.01 | <0.01 | <0.01 | <0.01 | 0.02 | <0.01 | 0.03 | 0.03 | <0.01 | <0.01 | <0.01 | <0.01 | 0.1 |
| S. enteritidis G14 | <0.01 | <0.01 | <0.01 | 0.02 | 0.02 | <0.01 | 0.02 | 0.03 | 0.02 | 0.01 | <0.01 | <0.01 | 0.1 |
| S. marcescens I AM 1184 | <0.01 | <0.01 | <0.01 | <0.01 | 0.8 | <0.01 | 0.03 | 0.1 | <0.01 | <0.01 | <0.01 | <0.01 | 0.03 |
| P. merganii IFO 3445 | <0.01 | <0.01 | 0.01 | <0.01 | <0.01 | 0.02 | 0.03 | 0.03 | <0.01 | <0.01 | <0.01 | <0.01 | 0.01 |
| P. vulgalis OX-19 | <0.01 | <0.01 | <0.01 | <0.01 | <0.01 | <0.01 | 0.02 | 0.02 | <0.01 | <0.01 | <0.01 | <0.01 | 0.01 |
| P. rettgeric IFO3850 | <0.01 | <0.01 | <0.01 | <0.01 | <0.01 | 0.02 | 0.03 | 0.02 | <0.01 | <0.01 | <0.01 | <0.01 | 0.01 |
| E. cloacae 963 | <0.01 | <0.01 | 0.03 | <0.01 | 0.05 | 0.05 | 0.1 | 0.1 | 0.02 | <0.01 | <0.01 | <0.01 | 0.1 |
| M. euteus ATCC 9341 | 0.1 | 0.8 | 0.2 | 0.8 | 1.6 | 0.8 | 0.8 | 1.6 | 1.6 | 1.6 | 0.01 | 0.2 | 1.6 |
| P. aeruginosa NCTC 1049 | 0.1 | 0.8 | 0.8 | 0.8 | 0.05 | 3.1 | 1.6 | 0.2 | 0.1 | 0.1 | 0.2 | 0.02 | 0.8 |
| B. subtilis ATCC 6633 | 0.1 | 1.6 | 0.4 | 1.6 | 1.6 | 1.6 | 1.6 | 0.3 | 0.8 | 0.8 | 0.4 | 0.4 | 3.1 |

Compound I:
(6R,7R)-7-[(Z)-2-(2-amino-1,3-thiazol-4-yl)-2-phenoxy-iminoacetamide]-3-(6,7-dihydroxy-2-isoquinolinium)methyl-3-cephem-4-carboxylate

Compound II:
(6R,7R)-7-[(Z)-2-(2-amino-1,3-thiazol-4-yl)-2-(o-chlorophenoxyimino)-acetamide]-3-(6,7-dihydroxy-2-isoquinolinium)methyl-3-cephem-4-carboxylate

Compound III:
(6R,7R)-7-[(Z)-2-(2-amino-1,3-thiazol-4-yl-2-(p-chlorophenoxyimino)acetamide]-3-(6,7-dihydroxy-2-isoquinolinium)methyl-3-cephem-4-carboxylate

Compound IV:
(6R,7R)-7-[(Z)-2-(2-amino-1,3-thiazol-4-yl)-2-(p-phenylphenoxy)iminoacetamide]-3-(6,7-dihydroxy-2-isoquinolinium)methyl-3-cephem-4-carboxylate

Compound V:
(6R,7R)-7-[(Z)-2-(2-amino-1,3-thiazol-4-yl-2-(p-carboxymethyl)phenoxyiminoacetamide]-3-(6,7-dihydroxy-2-isoquinolinium)methyl-3-cephem-4-carboxylate

Compound VI:
(6R,7R)-7-[(Z)-2-(2-amino-1,3-thiazol-4-yl)-2-phenoxyiminoacetamide]-3-(4-carboxy-6,7-dihydroxy-2-isoquinolinium)methyl-3-cephem-4-carboxylate

Compound VII:
(6R,7R)-7-[(Z)-2-(2-amino-1,3-thiazol-4-yl)-2-phenoxyiminoacetamide]-3-(1-chloro-4-carboxy-6,7-dihydroxy-2-isoquinolinium)methyl-3-cephem-4-carboxylate

Compound VIII:
(6R,7R)-7-[(Z)-2-(2-amino-1,3-thiazol-4-yl)-2-(m-carboxyphenoxyiminoacetamide]-3-(6,7-dihydroxy-2-isoquinoliniuim)methyl-3-cephem-4-carboxylate

Compound IX:

(6R,7R)-7-[(Z)-2-(2-amino-1,3-thiazol-4-yl)-2-phenoxyiminoacetamide]-3-(6,7-diacetoxy-2-iso quinolinium)methyl-3-cephem-4-carboxylate

Compound X:

(6R,7R)-7-[(Z)-2-(2-amino-1,3-thiazol-4-yl)-2-phenoxyiminoacetamide]-3-(6-acetoxy-7-hydroxy-2-isoquinolinium)methyl-3-cephem-4-carboxylate

Compound XI:

(6R,7R)-[(Z)-2(5-amino-1,2,4-thiadiazol-3-yl)-2-phenoxyiminoacetamide]-3-(6,7-dihydroxy-2-isoquinolinium)methyl-3-cephem-carboxylate

Comparative Compound A:

(6R,7R)-7-[(Z)-2-(2-amino-1,3-thiazol-4-yl)-2-(2-carboxypropyl-2-yloxyimino)acetamide]-3-(6,7-dihydroxy-2-isoquinolinium)methyl-3-cephem-4-carboxylate

CAZ (Ceftazidime)

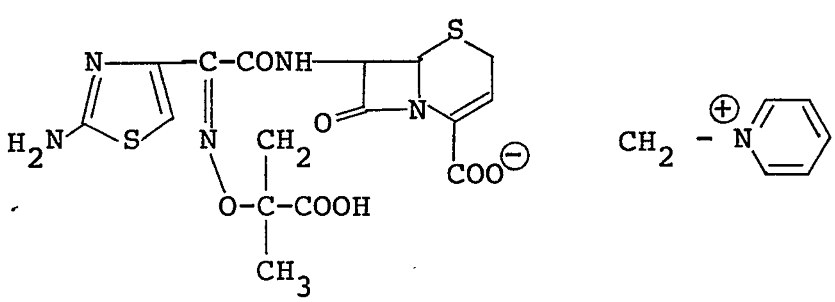

Referential Example (Comparative Compound A):

(6R,7R)-7-[(Z)-2-(2-amino-1,3-thiazol-4-yl-2-(2-carboxypropyl-2-oxyimino)acetamide-3-(6,7-dihydroxy-2-isoquinolinium)methyl-3-cephem-4-carboxylate

820 mg of 7-amino-3-(6,7-dihydroxy-2-isoquinolinium)methyl-3-cephem-4-carboxylate dihydrochloride was dissolved in 10 ml of N,N-dimethylacetamide, and 0.80 g

of triethylamine was added at -20°C. Meanwhile, 1.07 g of 2-tritylamino-1,3-thiazol-2-(2-t-butoxycarbonylpropyl-2-yloxyiminoacetic acid was reacted with 0.45 g of phosphorus pentachloride dissolved in 20 ml of dichloromethane at -10°C for 30 minutes. The solution was added to the above N,N-dimethylacetamide solution at -20°C. The temperature was gradually raised to room temperature, and stirring was conducted at room temperasture for about 1 hour. Afte the low-boiling solvent was distilled off from the reaction mixture under reduced pressure, the residue was dissolved in a solvent mixture of trifluoroacetic acid:water = 10:1, followed by stirring overnight at room temperature. The reaction mixture was dispersed in ether and precipitated. The precipitate was filtered, water-washed, then dissolved in a 0.5N hydrochloric acid aqueous solution, and eluted with a water/methanol solvent mixture using a CHP-20 resin column (reverted phase). From a fraction eluted at a ratio of methanol:water = about 20:8, 150 mg of the captioned compound was isolated.

NMR ($d_6$ - DMSO - $D_2$ O) :

3.5 2H (b)
5.22 1H (d)
5.5 2H (b)
5.92 1H (d)
6.71 1H (s)
7.45 1H (s)
7.6 1H (s)
8.20 1H (d)
8.35 1H (d)

## Claims

1. A cephalosporin compound represented by formula (I)

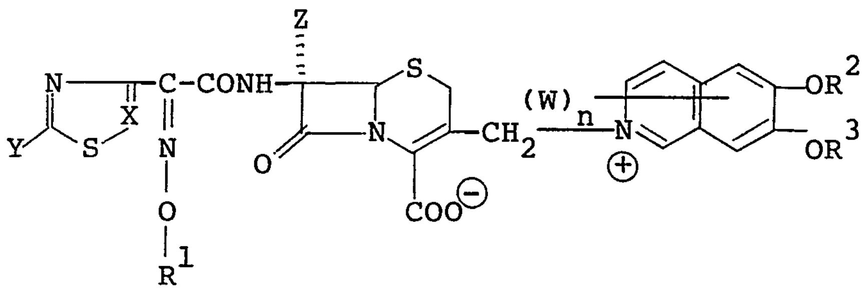

... (I)

wherein

X denotes -CH= or -N=,

Y denotes an amino group that may be protected,

Z denotes a hydrogen atom, a methoxy group or a formylamino group,

$R^1$ denotes an aromatic hydrocarbon group that may be substituted,

$R^2$ and $R^3$ are the same or different, and each denotes a hydrogen atom or a protective group of a hydroxyl group,

W denotes a substituent, and

n is an integer of 0 to 5,

or its salt.

2. A process for the production of a cephalosporin compound represented by formula (I)

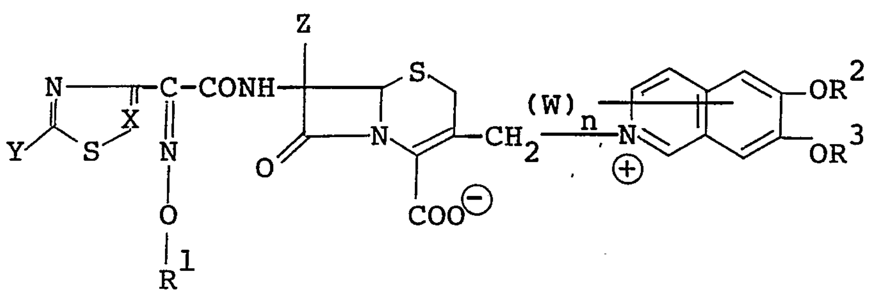

... (I)

wherein

X denotes -CH= or -N=,

Y denotes an amino group that may be protected,

Z denotes a hydrogen atom, a methoxy group or a formylamino group,

$R^1$ denotes an aromatic hydrocarbon group that may be substituted,

$R^2$ and $R^3$ are the same or different, and each denotes a hydrogen atom or a protective group of a hydroxyl group,

W denotes a substituent, and

n is an integer of 0 to 5,

or its salt, which comprises reacting a compound represented by formula (2)

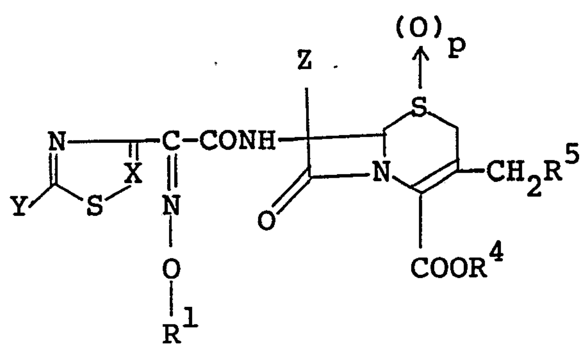

... (2)

wherein

X, Y, Z, $R^1$ are as defined above,

$R^5$ denotes a group that can be left by the reaction with an isoquinoline compound of formula (3) described below,

$R^4$ denotes a protective group of a carboxyl group, and

p is 0 or 1,

with an isoquinoline compound represented by formula (3)

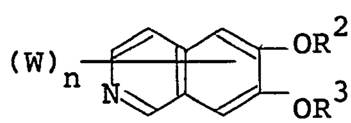

wherein $R^2$, $R^3$, W and n are as defined above, and if required, subjecting the reaction product to deprotection, reduction, esterification and salt formation.

3. A process for the production of a cephalosporin compound represented by formula (I)

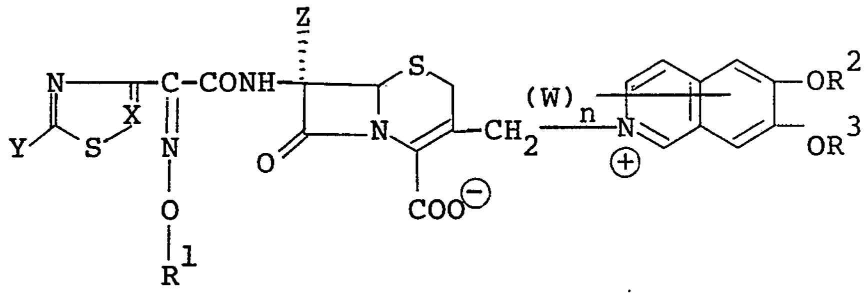

... (I)

wherein

X denotes -CH= or -N=,

Y denotes an amino group that may be protected,

Z denotes a hydrogen atom, a methoxy group or a formylamino group,

$R^1$ denotes an aromatic hydrocarbon group that may be substituted,

$R^2$ and $R^3$ are the same or different, and each denotes a hydrogen atom or a protective group of a hydroxyl group,

W denotes a substituent, and

n is an integer of 0 to 5,

or its salt, which comprises reacting a compound represented by formula (4)

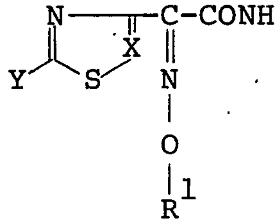

... (4)

wherein

X, Y and $R^1$ are as defined above,

its salt or its reactive derivative with a compound represented by formula (5)

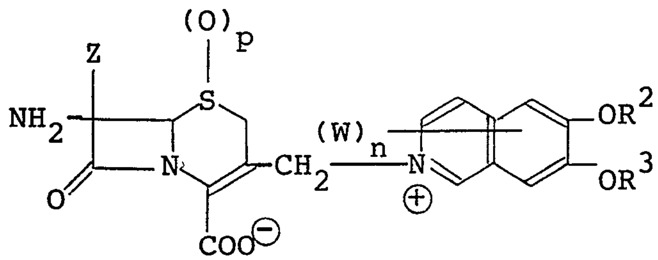

... (5)

wherein

Z, $R^2$, $R^3$, W and n are as defined above, and

p is 0 or 1,

its salt, its ester or its reactive derivative, and if required, subjecting the reaction product to deprotection, salt formation, esterification and reduction.

4. A process for the production of a cephalosporin compound represented by formula (I)

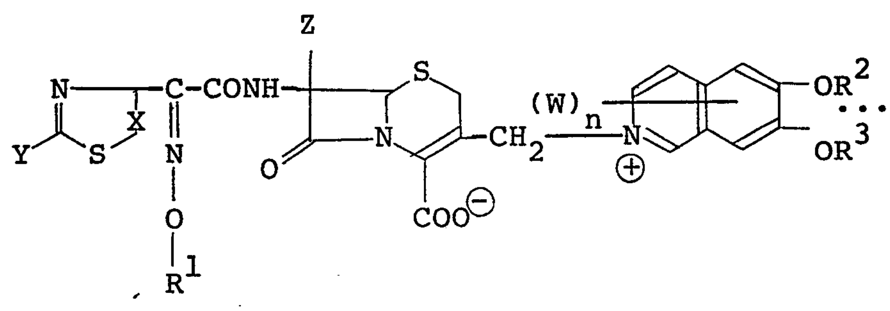

... (I)

wherein

X denotes -CH= or -N=,

Y denotes an amino group that may be protected,

Z denotes a hydrogen atom, a methoxy group or a formylamino group,

$R^1$ denotes an aromatic hydrocarbon group that may be substituted,

$R^2$ and $R^3$ are the same or different, and each denotes a hydrogen atom or a protective group of a hydroxyl group,

W denotes a substituent, and

n is an integer of 0 to 5,

or its salt, which comprises reacting a compound represented by formula (6)

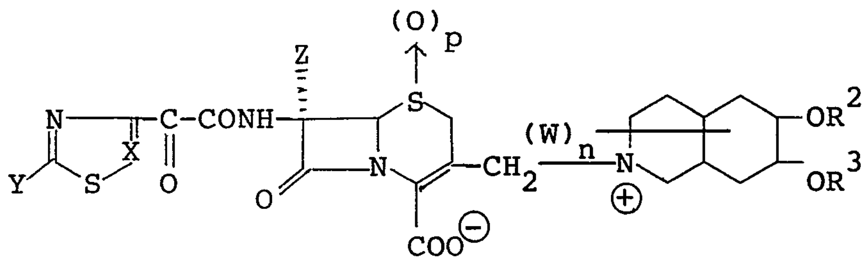 ... (6)

wherein

Y, Z, $R^2$, $R^3$, W and n are as defined above, and

p is 0 or 1,

its salt or its ester with a compound represented by formula (7)

$$NH_2-O-R^1 \quad \dots (7)$$

wherein

$R^1$ is as defined above,

its protected compound or its salt, and if required, subjecting the reaction product to deprotection, salt formation, esterification and reduction.

5. A process for the production of a cephalosporin compound represented by formula (1)

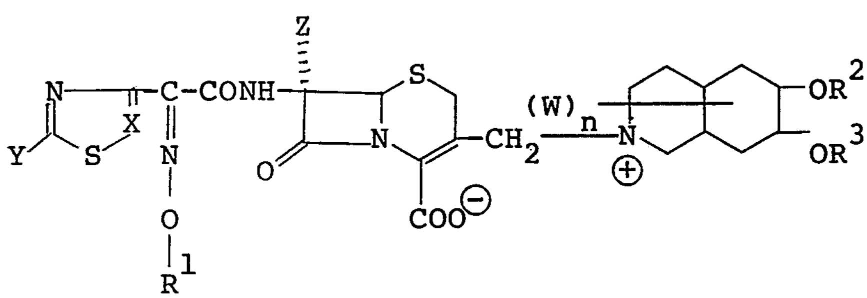 ... (I)

wherein

X denotes -CH= or -N=,

Y denotes an amino group that may be protected,

Z denotes a hydrogen atom, a methoxy group or a formylamino group,

$R^1$ denotes an aromatic hydrocarbon group that may be substituted,

$R^2$ and $R^3$ are the same or different, and each denotes a hydrogen atom or a protective group of a hydroxyl group,

W denotes a substituent, and

n is an integer of 0 to 5,

or its salt, which comprises reacting a compound represented by formula I-(1)

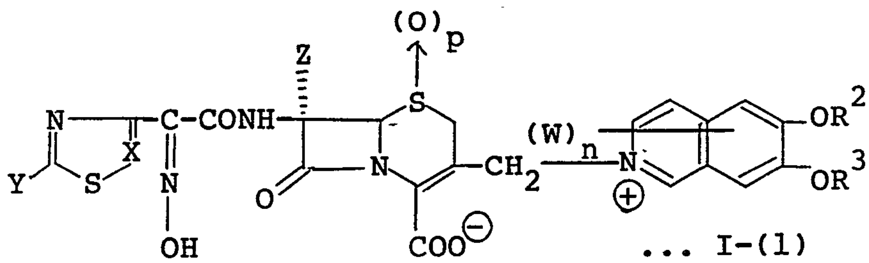

... I-(1)

wherein

X, Y, Z, $R^2$, $R^3$, W and n are as defined above,

and p is 0 or 1,

its salt or its ester with a compound represented by formula (10)

$$Hal - R^1 \quad ... (10)$$

wherein

$R^1$ is as defined above, and

Hal denotes a halogen atom,

and if required, subjecting the reaction product to deprotection, salt formation, esterification and reduction.

6. A pharmaceutical composition comprising an effective amount of the cephalosporin compound represented by formula (1) or its salt and a pharmaceutically acceptable inactive carrier.

# INTERNATIONAL SEARCH REPORT

International Application No PCT/JP88/00089

**I. CLASSIFICATION OF SUBJECT MATTER** (if several classification symbols apply, indicate all) [6]

According to International Patent Classification (IPC) or to both National Classification and IPC

Int.Cl[4] C07D501/46, 501/57, A61K31/545

**II. FIELDS SEARCHED**

Minimum Documentation Searched [7]

| Classification System [8] | Classification Symbols |
|---|---|
| IPC | C07D501/46, 501/57, A61K31/545 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | JP, A, 58-57386 (Eli Lilly and Company) 5 April 1983 (05. 04. 83) Page 3, left column, line 20 to page 4, left column, line 19 & US, A, 4,396,619 & DE, A, 3,233,377 & FR, A, 2,512,448 | 1-6 |
| X | JP, A, 59-130294 (Hoechst A.G.) 26 July 1984 (26. 07. 84) Claims 1 to 7, page 17, Example 46 & DE, A, 3,247,614 & EP, A, 111,935 | 1-6 |
| A | JP, A, 51-149296 (Takeda Chemical Industries, Ltd.) 8 June 1976 (08. 06. 76) Claim 3, page 7, right column, line 19 to right column, 4th line from the bottom & GB, A, 1,536,281 & DE, A, 2,556,736 | 1-6 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| April 18, 1988 (18. 04. 88) | May 2, 1988 (02. 05. 88) |
| **International Searching Authority** | **Signature of Authorized Officer** |
| Japanese Patent Office | |